# EUROPEAN PATENT APPLICATION

(11) **EP 4 763 062 A1**
(43) Date of publication of application: **24.06.2026**
(21) Application number: 24911572.6
(22) Date of filing: 28.12.2024
(51) Int. Cl.: A61B 5/02, G06F 1/3206

(54) **DEVICE ACTIVATION METHOD AND CIRCUIT, AND ELECTRONIC DEVICE**

(30) Priority: 29.12.2023 CN 202311872475
(71) Applicant: HUAWEI TECHNOLOGIES CO., LTD., Shenzhen 518129 (CN)
(72) Inventor: LIU, Xiangyu, Shenzhen, Guangdong 518129 (CN)
(74) Representative: Maiwald GmbH
(86) International application number: PCT/CN2024/143470
(87) International publication number: WO 2025/140658

(57) **Abstract**

This application discloses a device activation method, a circuit, and an electronic device. The electronic device is configured to measure a body indicator of a user. The electronic device includes an activation circuit, a power management module, a battery, and a data processing module. A first end of the activation circuit is connected to the power management module, a second end is connected to the battery, and the power management module is connected to the data processing module. The data processing module is configured to determine the body indicator of the user based on data obtained by the electronic device. The activation circuit is configured to detect whether the user wears the electronic device or whether the user prepares to wear the electronic device. When detecting that the user wears the electronic device or the user prepares to wear the electronic device, the activation circuit switches a connection between the first end and the second end from being cut off to being conducted. The battery may be configured to supply power to the data processing module by using the battery management module after the connection between the first end and the second end is conducted. It can be learned that, based on the method, seamless activation of the device can be implemented by using the activation circuit without requiring user manual activation, thereby simplifying user's operations.

## Description

This application claims priority to Chinese Patent Application No. 202311872475.5, filed with the China National Intellectual Property Administration on December 29, 2023 and entitled "DEVICE ACTIVATION METHOD, CIRCUIT, AND ELECTRONIC DEVICE", which is incorporated herein by reference in its entirety.

### TECHNICAL FIELD

This application relates to the field of terminals, and in particular, to a device activation method, a circuit, and an electronic device.

### BACKGROUND

With continuous progress of science and technology, a growing number of devices are being designed to be worn on users, to measure body indicators of the users such as blood glucose, heart rate, body temperature, blood pressure, blood lipid, and blood ketone. For example, common devices include continuous glucose monitoring (continuous glucose monitoring, CGM) devices, which can be used to measure users' blood glucose, continuous ketone monitoring (continuous ketone monitoring, CKM) devices, which can be used to measure users' blood ketone, continuous lactate monitoring (continuous lactate monitoring, CLM) devices, which can be used to measure users' lactate, and electrocardiogram patches, which are used to measure users' electrocardiogram signals.

However, for such devices that need to be worn on users to measure their body indicators, users need to first actively activate the devices to power on the device before wearing. Only then the devices can measure the body indicators once worn. This process is cumbersome and cannot achieve seamless activation.

### SUMMARY

This application provides a device activation method, a circuit, and an electronic device. Based on the method, it can be detected that a user wears the electronic device or prepares to wear the electronic device, to implement seamless activation of the device, thereby facilitating an operation of the user.

According to a first aspect, an embodiment of this application provides a first electronic device, where the first electronic device is configured to measure a body indicator of a user, the first electronic device includes an activation circuit, a power management module, a battery, and a data processing module, the activation circuit includes a first end and a second end, the first end is connected to the power management module, the second end is connected to the battery, and the power management module is connected to the data processing module; the activation circuit is configured to determine whether the user wears the first electronic device or whether the user prepares to wear the first electronic device; when the activation circuit determines that the user wears the first electronic device or the user prepares to wear the first electronic device, a connection between the first end and the second end is switched from being cut off to being conducted; the battery is configured to supply power to the data processing module by using the power management module after the connection between the first end and the second end is conducted; and the data processing module is configured to determine the body indicator of the user based on data obtained by the first electronic device.

Based on the first electronic device provided in this embodiment of this application, the activation circuit can be used to detect whether the user wears the electronic device or prepares to wear the electronic device, and when detecting that the user wears the electronic device or prepares to wear the electronic device, automatically activates the device, that is, power is supplied to a component in the device, to reduce operation complexity when the user activates the device, thereby implementing seamless activation of the device.

It may be understood that the activation circuit may alternatively exist separately, and does not need to be located in the first electronic device.

With reference to the first aspect, in an implementation, the data processing module, when powered on, is further configured to control the connection between the first end and the second end of the activation circuit to remain continuously conductive.

After the battery supplies power to the data processing module by using the power management module, the data processing module may be in a power-on state, and the data processing module may reversely control the activation circuit, so that the activation circuit can continuously maintain a state in which the battery supplies power to the data processing module. In this way, it can be ensured that the electronic device can continuously maintain an active state after being activated, and activation of the device is prevented from being intentionally or accidentally triggered by the user in a use process.

With reference to the first aspect, in an implementation, the activation circuit includes a first MOS transistor and a first sensor, where a drain of the first MOS transistor is the first end, a source of the first MOS transistor is the second end, one end of the first sensor is connected to a gate of the first MOS transistor, and the other end of the first sensor is grounded; the first sensor is configured to detect whether the user wears the first electronic device or whether the user prepares to wear the first electronic device; the first sensor switches from being cut off to being conducted when detecting that the user wears the first electronic device or the user prepares to wear the first electronic device; and when the first sensor is cut off, the first MOS transistor is cut off, and when the first sensor is conducted, the first MOS transistor is conducted.

In other words, a characteristic that the sensor can sense an environmental change may be cleverly used, the sensor is added to the activation circuit, the sensor is used to detect actions of the user wearing and preparing to wear the electronic device, and a switching characteristic of the MOS transistor is used, to implement device activation in cooperation with the sensor.

With reference to the first aspect, in an implementation, the activation circuit further includes a first resistor, where one end of the first resistor is connected to the source of the first MOS transistor, and the other end of the first resistor is connected to the gate of the first MOS transistor.

When the resistor is added to the activation circuit, a leakage current generated in the activation circuit can be effectively reduced.

With reference to the first aspect, in an implementation, the activation circuit, the power management module, the battery, and the data processing module are all located in a first component of the first electronic device, the first component is located within a second component, and the first component is detached from the second component when the user wears the first electronic device; the first sensor is an optical sensor, the first sensor is disposed on a side facing the second component when the first component is located within the second component, and the first sensor is configured to sense changes in illumination; and the first sensor switches from being cut off to being conducted when sensing that light intensity is greater than a first threshold.

The first sensor may sense that the light intensity is greater than the first threshold when the first component is detached from the second component.

In some implementations, the first component may be a transmitter, and the second component may be an implanter.

It can be learned that, in this embodiment of this application, when the user wears the first electronic device, an action of detaching the first component from the second component exists. When the first component is detached from the second component, a process in which a side facing the second component in the first component changes from receiving no light to receiving light occurs. Therefore, the optical sensor is added to detect the action of wearing the electronic device by the user, to effectively and accurately control an occasion when the user wears the electronic device, thereby activating the device when the user wears the electronic device.

With reference to the first aspect, in an implementation, the activation circuit, the power management module, the battery, and the data processing module are all located in a first component of the first electronic device, the first component is covered by a third component, the third component is opaque, and the user removes the third component when preparing to wear the first electronic device; the first sensor is an optical sensor, the first sensor is disposed on a side facing the third component, and the first sensor is configured to sense changes in illumination; and the first sensor switches from being cut off to being conducted when sensing that light intensity is greater than a first threshold.

The first sensor may sense that the light intensity is greater than the first threshold when the user removes the third component.

In some implementations, the first component may be a transmitter, and the third component may be a packaging cover.

It can be learned that, in this embodiment of this application, when the user prepares to wear the first electronic device, an action of removing the third component covering the first component exists. When the user removes the third component, a process in which a side facing the third component in the first component changes from receiving no light to receiving light occurs. Therefore, the optical sensor is added to detect the action of preparing to wear the electronic device by the user, to effectively and accurately control an occasion when the user prepares to wear the electronic device, thereby activating the device when the user prepares to wear the electronic device.

With reference to the first aspect, in an implementation, the activation circuit, the power management module, the battery, and the data processing module are all located in a first component of the first electronic device, the first component is located within a second component, and the first component is detached from the second component when the user wears the first electronic device; the first sensor is a magnetic sensor, the second component includes a magnet, and the first sensor is configured to sense changes in a magnetic field; and the first sensor switches from being cut off to being conducted when sensing that magnetic field strength of the magnet is less than a second threshold.

The first sensor may sense that the magnetic field strength of the magnet is less than the second threshold when the first component is detached from the second component.

In some implementations, the first component may be a transmitter, and the second component may be an implanter.

It can be learned that, in this embodiment of this application, when the user wears the first electronic device, an action of detaching the first component from the second component exists. When the magnet is disposed in the second component, and when the first component is detached from the second component, a change process in which the first component is far away from magnetic field effect of the magnet occurs. Therefore, the magnetic sensor is added to detect the action of wearing the electronic device by the user, to effectively and accurately control an occasion when the user wears the electronic device, thereby activating the device when the user wears the electronic device.

With reference to the first aspect, in an implementation, the activation circuit, the power management module, the battery, and the data processing module are all located in a first component of the first electronic device, the first component is covered by a third component, and the user removes the third component when preparing to wear the first electronic device; the first sensor is a magnetic sensor, the third component includes a magnet, and the first sensor is configured to sense changes in a magnetic field; and the first sensor switches from being cut off to being conducted when sensing that magnetic field strength of the magnet is less than a second threshold.

The first sensor may sense that the magnetic field strength of the magnet is less than the second threshold when the user removes the third component.

In some implementations, the first component may be a transmitter, and the third component may be a packaging cover.

It can be learned that, in this embodiment of this application, when the user prepares to wear the first electronic device, an action of removing the third component covering the first component exists. When the magnet is disposed in the third component, and when the user removes the third component, a change process in which the first component is far away from magnetic field effect of the magnet occurs. Therefore, the magnetic sensor is added to detect the action of preparing to wear the electronic device by the user, to effectively and accurately control an occasion when the user wears the electronic device, thereby activating the device when the user prepares to wear the electronic device.

With reference to the first aspect, in an implementation, the magnetic sensor is any one of an anisotropic magnetoresistance AMR sensor, a tunnel magnetoresistance TMR sensor, a giant magnetoresistance GMR sensor, a magnetic reed switch, or a Hall switch.

With reference to the first aspect, in an implementation, the activation circuit further includes a second MOS transistor, a gate of the second MOS transistor is connected to the data processing module, a source of the second MOS transistor is grounded, and a drain of the second MOS transistor is connected to the gate of the first MOS transistor; the data processing module, when powered on, outputs a first voltage to the gate of the second MOS transistor; and when the gate of the second MOS transistor receives the first voltage, the second MOS transistor switches from being cut off to being conducted, so that the first MOS transistor is continuously conducted.

The first voltage is greater than a voltage originally output by the data processing module to the gate of the second MOS transistor, or further, is greater than a voltage originally output by the data processing module to the gate of the second MOS transistor and reaches a specific threshold.

It can be learned that, after the data processing module is powered on, the MOS transistor in the activation circuit may be reversely controlled, so that a status of connection from the first end to the second end of the activation circuit is no longer affected by the first sensor. In this way, the device can continuously maintain an active state after being activated.

With reference to the first aspect, in an implementation, the power management module includes a power management unit PMU, and the data processing module includes any one of a microcontroller unit MCU, a digital signal processor, an ARM processor, a field programmable gate array FPGA, or an application-specific integrated circuit ASIC.

With reference to the first aspect, in an implementation, the first electronic device further includes a communication module. The communication module is configured to send a broadcast signal to surroundings after the data processing module is powered on, where the broadcast signal is used to request to establish a communication connection; and the communication module is further configured to: receive information that is sent by a second electronic device and that is used to agree to establish the communication connection, and establish the communication connection to the second electronic device.

In other words, after being activated, the first electronic device may actively send a broadcast signal to surroundings, to seek to establish a communication connection to a surrounding device. In this way, operations performed by the user when the user initiates establishment of the communication connection by using the second electronic device when needing to establish the communication connection between the first electronic device and the second electronic device can be reduced, and a speed of establishing the communication connection between the first electronic device and the second electronic device can be accelerated.

With reference to the first aspect, in an implementation, the activation circuit is configured to detect whether the user wears the first electronic device, and the first electronic device further includes a timing module and a communication module, where the timing module is configured to start timing after the data processing module is powered on, where duration of timing of the first electronic device is a length of time elapsed since the user started wearing the first electronic device; and the communication module is configured to: send the data of the body indicator to a second electronic device after establishing a communication connection to the second electronic device, and send, to the second electronic device, first duration obtained by the first electronic device through timing, where the first duration is related to a first time, and the first time is a time at which the second electronic device starts to display the data of the body indicator.

After being worn on the user, the first electronic device usually needs to wait for a period of initialization time before the first electronic device can obtain stable data that is of the body indicator and that can reflect a real status of the user. Therefore, after establishing the communication connection to the first electronic device, the second electronic device usually needs to wait for the initialization time to end before starting to display the data of the body indicator measured by the first electronic device.

Because the activation circuit provided in this embodiment of this application can implement device activation when the user wears the first electronic device, the first electronic device can accurately learn a time point at which the user wears the first electronic device, and accurately record duration experienced from a time when the user starts to wear the first electronic device. In this way, when the second electronic device needs to display the data of the body indicator measured by the first electronic device, the second electronic device can accurately calculate, based on the duration recorded by the first electronic device, when the second electronic device can output stable data that can reflect a real status of the user, to prevent the user from waiting for an excessively long time when viewing the data of the body indicator by using the second electronic device.

With reference to the first aspect, in an implementation, the activation circuit is configured to detect whether the user prepares to wear the first electronic device, and the first electronic device further includes a timing module, a communication module, and a second sensor; the second sensor may be configured to collect the data used to reflect the body indicator after the data processing module is powered on; and the timing module may be configured to start timing when the data collected by the second sensor meets a first preset condition, where duration of timing of the first electronic device is a length of time elapsed since the user started wearing the first electronic device; and the communication module is configured to: send the data of the body indicator to a second electronic device after establishing a communication connection to the second electronic device, and send, to the second electronic device, first duration obtained by the first electronic device through timing, where the first duration is related to a first time, and the first time is a time at which the second electronic device starts to display the data of the body indicator.

Because the activation circuit provided in this embodiment of this application can implement device activation before the user wears the first electronic device, after being activated, the first electronic device may alternatively determine, based on the data collected by the sensor that can monitor the body indicator of the user, a time point at which the user wears the first electronic device, and accurately record duration experienced from a time when the user starts to wear the first electronic device. In this way, when the second electronic device needs to display the data of the body indicator measured by the first electronic device, the second electronic device can accurately calculate, based on the duration recorded by the first electronic device, when the second electronic device can output stable data that can reflect a real status of the user, to prevent the user from waiting for an excessively long time when viewing the data of the body indicator by using the second electronic device.

With reference to the first aspect, in an implementation, the activation circuit is configured to detect whether the user prepares to wear the first electronic device, and the first electronic device further includes a second sensor and an output module; the second sensor is configured to collect the data used to reflect the body indicator after the data processing module is powered on; the output module is configured to output first prompt information when the data collected by the second sensor meets a second preset condition, where the first prompt information prompts the user to wear the first electronic device in a timely manner; and the output module is further configured to output second prompt information when the data collected by the second sensor meets a third preset condition, where the second prompt information prompts a wear failure of the first electronic device to the user.

In other words, the first electronic device may detect, based on the data collected by the second sensor, a state of wearing the first electronic device by the user, to remind the user to wear the first electronic device in a timely manner or remind the user of a wear failure, thereby providing a more user-friendly wearing service for the user.

In some implementations, if a communication connection is established between the first electronic device and the second electronic device, when the data collected by the second sensor meets the second preset condition, the first electronic device may send the first prompt information to the second electronic device, and the second electronic device then outputs the first prompt information. In this way, the user can view, by using the second electronic device, the prompt information that reminds the user to wear the first electronic device in a timely manner.

In some implementations, if a communication connection is established between the first electronic device and the second electronic device, when the data collected by the second sensor meets the third preset condition, the first electronic device may send the second prompt information to the second electronic device, and the second electronic device then outputs the second prompt information. In this way, the user can view, by using the second electronic device, the prompt information that prompts the user that the first electronic device fails to be worn.

In some implementations, the first electronic device is a continuous glucose monitoring CGM device, and the body indicator is blood glucose; or the first electronic device is a continuous ketone monitoring CKM device, and the body indicator is blood ketone; or the first electronic device is a continuous lactate monitoring CLM device, and the body indicator is lactate; or the first electronic device is an electrocardiogram patch, and the body indicator is an electrocardiogram signal.

According to a second aspect, an embodiment of this application provides a device activation method, where the method is applied to a first electronic device, the first electronic device is configured to measure a body indicator of a user, the first electronic device includes an activation circuit, a power management module, a battery, and a data processing module, the activation circuit includes a first end and a second end, the first end is connected to the power management module, the second end is connected to the battery, and the power management module is connected to the data processing module, and the method includes: The first electronic device determines, by using the activation circuit, whether the user wears the first electronic device or whether the user prepares to wear the first electronic device; the first electronic device switches, by using the activation circuit when determining that the user wears the first electronic device or the user prepares to wear the first electronic device, a connection between the first end and the second end from being cut off to being conducted; the first electronic device enables, after the connection between the first end and the second end is conducted, the battery to supply power to the data processing module by using the power management module; and the first electronic device determines, by using the data processing module, the body indicator of the user based on data obtained by the first electronic device.

Based on the method provided in the second aspect, the activation circuit can be used to detect whether the user wears the electronic device or prepares to wear the electronic device, and when detecting that the user wears the electronic device or prepares to wear the electronic device, automatically activates the device, that is, power is supplied to a component in the device, to reduce operation complexity when the user activates the device, thereby implementing seamless activation of the device.

With reference to the second aspect, in an implementation, the method further includes: The first electronic device controls the connection between the first end and the second end of the activation circuit to be continuously conducted in a power-on condition of the data processing module.

With reference to the second aspect, in an implementation, the activation circuit includes a first MOS transistor and a first sensor, where a drain of the first MOS transistor is the first end, a source of the first MOS transistor is the second end, one end of the first sensor is connected to a gate of the first MOS transistor, and the other end of the first sensor is grounded; that the first electronic device detects, by using the activation circuit, whether the user wears the first electronic device or whether the user prepares to wear the first electronic device specifically includes: The first electronic device detects, by using the first sensor, whether the user wears the first electronic device or whether the user prepares to wear the first electronic device; the first sensor switches from being cut off to being conducted when detecting that the user wears the first electronic device or the user prepares to wear the first electronic device; and when the first sensor is cut off, the first MOS transistor is cut off, and when the first sensor is conducted, the first MOS transistor is conducted.

With reference to the second aspect, in an implementation, the activation circuit further includes a first resistor, where one end of the first resistor is connected to the source of the first MOS transistor, and the other end of the first resistor is connected to the gate of the first MOS transistor.

With reference to the second aspect, in an implementation, the activation circuit, the power management module, the battery, and the data processing module are all located in a first component of the first electronic device, the first component is located within a second component, and the first component is detached from the second component when the user wears the first electronic device; the first sensor is an optical sensor, and the first sensor is disposed on a side facing the second component when the first component is located within the second component; and that the first electronic device detects, by using the first sensor, whether the user wears the first electronic device specifically includes: The first electronic device detects, based on an illumination change sensed by the first sensor, whether the user wears the first electronic device; and the first sensor switches from being cut off to being conducted when sensing that light intensity is greater than a first threshold.

With reference to the second aspect, in an implementation, the activation circuit, the power management module, the battery, and the data processing module are all located in a first component of the first electronic device, the first component is covered by a third component, the third component is opaque, and the user removes the third component when preparing to wear the first electronic device; the first sensor is an optical sensor, and the first sensor is disposed on a side facing the third component; and that the first electronic device detects, by using the first sensor, whether the user prepares to wear the first electronic device specifically includes: The first electronic device detects, based on an illumination change sensed by the first sensor, whether the user prepares to wear the first electronic device; and the first sensor switches from being cut off to being conducted when sensing that light intensity is greater than a first threshold.

With reference to the second aspect, in an implementation, the activation circuit, the power management module, the battery, and the data processing module are all located in a first component of the first electronic device, the first component is located within a second component, and the first component is detached from the second component when the user wears the first electronic device; the first sensor is a magnetic sensor, and the second component includes a magnet; and that the first electronic device detects, by using the first sensor, whether the user wears the first electronic device specifically includes: The first electronic device detects, based on changes in a magnetic field sensed by the first sensor, whether the user wears the first electronic device; and the first sensor switches from being cut off to being conducted when sensing that magnetic field strength of the magnet is less than a second threshold.

With reference to the second aspect, in an implementation, the activation circuit, the power management module, the battery, and the data processing module are all located in a first component of the first electronic device, the first component is covered by a third component, and the user removes the third component when preparing to wear the first electronic device; the first sensor is a magnetic sensor, and the third component includes a magnet; and that the first electronic device detects, by using the first sensor, whether the user prepares to wear the first electronic device specifically includes: The first electronic device detects, based on changes in a magnetic field sensed by the first sensor, whether the user prepares to wear the first electronic device; and the first sensor switches from being cut off to being conducted when sensing that magnetic field strength of the magnet is less than a second threshold.

With reference to the second aspect, in an implementation, the activation circuit further includes a second MOS transistor, a gate of the second MOS transistor is connected to the data processing module, a source of the second MOS transistor is grounded, and a drain of the second MOS transistor is connected to the gate of the first MOS transistor, and after that the first electronic device enables the battery to supply the power to the data processing module by using the power management module, the method further includes: when the data processing module powered on, the first electronic device outputs a high voltage to the gate of the second MOS transistor using the data processing module; and when the gate of the second MOS transistor receives the high voltage, the first electronic device enables, by using the second MOS transistor, the first MOS transistor to be continuously conducted, so that the second MOS transistor switches from being cut off to being conducted when the gate of the second MOS transistor receives the high voltage.

With reference to the second aspect, in an implementation, after that the first electronic device enables the battery to supply power to the data processing module by using the power management module, the method further includes: The first electronic device sends a broadcast signal to surroundings, where the broadcast signal is used to request to establish a communication connection; the first electronic device receives information that is sent by a second electronic device and that is used to agree to establish the communication connection; and the first electronic device establishes the communication connection to the second electronic device.

With reference to the second aspect, in an implementation, the first electronic device detects, by using the activation circuit, whether the user wears the first electronic device, and after that the first electronic device enables the battery to supply the power to the data processing module by using the power management module, the method further includes: The first electronic device starts timing, where duration of timing of the first electronic device is a length of time elapsed since the user started wearing the first electronic device; and the first electronic device sends the data of the body indicator to a second electronic device after establishing a communication connection to the second electronic device, and sends, to the second electronic device, first duration obtained by the first electronic device through timing, where the first duration is related to a first time, and the first time is a time at which the second electronic device starts to display the data of the body indicator.

With reference to the second aspect, in an implementation, the first electronic device further includes a second sensor, the first electronic device detects, by using the activation circuit, whether the user prepares to wear the first electronic device, and after that the first electronic device enables the battery to supply the power to the data processing module by using the power management module, the method further includes: The first electronic device collects, by using the second sensor, the data used to reflect the body indicator; the first electronic device starts timing when the data collected by the second sensor meets a first preset condition, where duration of timing of the first electronic device is a length of time elapsed since the user started wearing the first electronic device; and the first electronic device sends the data of the body indicator to a second electronic device after establishing a communication connection to the second electronic device, and sends, to the second electronic device, first duration obtained by the first electronic device through timing, where the first duration is related to a first time, and the first time is a time at which the second electronic device starts to display the body indicator.

With reference to the second aspect, in an implementation, the first electronic device detects, by using the activation circuit, whether the user prepares to wear the first electronic device, the first electronic device further includes a second sensor, and the method further includes: The first electronic device collects, by using the second sensor, the data used to reflect the body indicator after the data processing module is powered on; the first electronic device outputs first prompt information when the data collected by the second sensor meets a second preset condition, where the first prompt information prompts the user to wear the first electronic device in a timely manner; and the first electronic device outputs second prompt information when the data collected by the second sensor meets a third preset condition, where the second prompt information prompts a wear failure of the first electronic device to the user.

With reference to the second aspect, in an implementation, the first electronic device is a continuous glucose monitoring CGM device, and the body indicator is blood glucose; or the first electronic device is a continuous ketone monitoring CKM device, and the body indicator is blood ketone; or the first electronic device is a continuous lactate monitoring CLM device, and the body indicator is lactate; or the first electronic device is an electrocardiogram patch, and the body indicator is an electrocardiogram signal.

According to a third aspect, an embodiment of this application provides a device activation method, where the method is applied to a first electronic device and a second electronic device, the first electronic device is configured to measure a body indicator of a user, the first electronic device includes an activation circuit, a power management module, a battery, a data processing module, and a first sensor, the activation circuit includes a first end and a second end, the first end is connected to the power management module, the second end is connected to the battery, the power management module is connected to the data processing module, and the data processing module is configured to determine the body indicator of the user based on data obtained by the first electronic device. The method includes: The first electronic device determines, by using the activation circuit, whether the user wears the first electronic device; the first electronic device switches, by using the activation circuit when detecting that the user wears the first electronic device, a connection between the first end and the second end from being cut off to being conducted; the first electronic device enables, after the connection between the first end and the second end is conducted, the battery to supply power to the data processing module by using the power management module; the first electronic device starts timing, where duration of timing of the first electronic device is a length of time elapsed since the user started wearing the first electronic device; the first electronic device sends, to the second electronic device after establishing a communication connection to the second electronic device, first duration of timing of the first electronic device; and after countdown for second duration, the second electronic device outputs a value that is of the body indicator and that is collected by the first electronic device in real time, where the second duration is determined based on first preset duration and the first duration.

The first preset duration is duration from a time when the first electronic device starts to measure the body indicator of the user to a time when the first electronic device collects stable data that can reflect a real body indicator of the user. The second duration is equal to the first preset duration minus the first duration.

Based on the method provided in the third aspect, the first electronic device may detect, by using the activation circuit, whether the user wears the first electronic device, so that the first electronic device is activated when the user wears the first electronic device. In addition, the first electronic device may start timing when being activated, so that the second electronic device determines, based on the duration of timing of the first electronic device, when to display the data of the body indicator collected by the first electronic device, so that it can be ensured that the body indicator displayed by the second electronic device to the user is a stable and accurate value, and a waiting time of the user can be reduced.

According to a fourth aspect, an embodiment of this application provides a device activation method, where the method is applied to a first electronic device and a second electronic device, the first electronic device is configured to measure a body indicator of a user, the first electronic device includes an activation circuit, a power management module, a battery, a data processing module, and a first sensor, the activation circuit includes a first end and a second end, the first end is connected to the power management module, the second end is connected to the battery, the power management module is connected to the data processing module, and the data processing module is configured to determine the body indicator of the user based on data obtained by the first electronic device. The method includes: The first electronic device determines, by using the activation circuit, whether the user prepares to wear the first electronic device; the first electronic device switches, by using the activation circuit when detecting that the user prepares to wear the first electronic device, a connection between the first end and the second end from being cut off to being conducted; the first electronic device enables, after the connection between the first end and the second end is conducted, the battery to supply power to the data processing module by using the power management module; the first electronic device collects, by using a second sensor, the data used to reflect the body indicator; the first electronic device starts timing when the data collected by the second sensor meets a preset condition, where duration of timing of the first electronic device is a length of time elapsed since the user started wearing the first electronic device; the first electronic device sends, to the second electronic device after establishing a communication connection to the second electronic device, first duration of timing of the first electronic device; and after countdown for second duration, the second electronic device outputs a value that is of the body indicator and that is collected by the first electronic device in real time, where the second duration is equal to initialization duration minus the first duration, and the initialization duration is duration from a time when the first electronic device starts to measure the body indicator of the user to a time when the first electronic device collects a stable value that can reflect a real body indicator of the user.

The first preset duration is the time taken by the first electronic device from the start of measuring the body indicator of the user to the moment the first electronic device collects stable data that can reflect the real body indicator of the user. The second duration is equal to the first preset duration minus the first duration.

Based on the method provided in the fourth aspect, the first electronic device may detect, by using the activation circuit, whether the user prepares to wear the first electronic device, so that the first electronic device is activated when the user prepares to wear the first electronic device. In addition, the first electronic device may monitor, by using the sensor after being activated, the action of wearing the electronic device by the user, to start timing when the user wears the electronic device, so that the second electronic device determines, based on the duration of timing of the first electronic device, when to display the data of the body indicator collected by the first electronic device, so that can be ensured that the body indicator displayed by the second electronic device to the user is a stable and accurate value, and a waiting time of the user can be reduced.

According to a fifth aspect, an embodiment of this application provides an electronic device, including a first electronic device, a second component, and/or a third component, where if the electronic device includes the first electronic device and the second component, a first component of the first electronic device is located in the second component; if the electronic device includes the first electronic device and the third component, the third component covers the first component in the first electronic device; or if the electronic device includes the first electronic device, the second component, and the third component, the first component of the first electronic device is located in the second component, and the third component covers a transmission outlet of the second component and is configured to cover the first component, where the first electronic device is the first electronic device in any one of the first aspect or the implementations of the first aspect.

For example, the first component may be a transmitter, the second component may be an implanter, and the third component may be a packaging cover.

According to a sixth aspect, an embodiment of this application provides an electronic device, including a memory, one or more processors, and one or more programs. When the one or more processors execute the one or more programs, the electronic device is enabled to implement the method according to any one of the second aspect or the implementations of the second aspect.

According to a seventh aspect, an embodiment of this application provides a computer-readable storage medium, including instructions. When the instructions are run on an electronic device, the electronic device is enabled to perform the method according to any one of the second aspect or the possible implementations of the second aspect.

According to an eighth aspect, an embodiment of this application provides a computer program product. When the computer program product is run on a computer, the computer is enabled to perform the method according to any one of the second aspect or the possible implementations of the second aspect.

For descriptions of beneficial effects in the second aspect to the eighth aspect, refer to descriptions of beneficial effects in the first aspect.

### BRIEF DESCRIPTION OF DRAWINGS

FIG. 1 is a diagram of a structure of an electronic device 100 according to an embodiment of this application;
FIG. 2 is a diagram of an operation of using an electronic device 100 according to an embodiment of this application;
FIG. 3 is a diagram of a structure of a device activation circuit 110 according to an embodiment of this application;
FIG. 4 is a multi-angle diagram of a transmitter in an electronic device 100 according to an embodiment of this application;
FIG. 5 is a diagram of a circuit structure of a device activation circuit 110 according to an embodiment of this application;
FIG. 6 is a diagram of a working principle of a device activation circuit 110 according to an embodiment of this application;
FIG. 7 is a diagram of a circuit structure of another device activation circuit 110 according to an embodiment of this application;
FIG. 8 is a schematic flowchart of a device activation method according to an embodiment of this application;
FIG. 9A to FIG. 9D are related user interfaces on an electronic device 200 according to an embodiment of this application;
FIG. 10 is a blood glucose change curve collected by an electronic device 100 after the electronic device 100 is worn on a user according to an embodiment of this application;
FIG. 11 is a diagram of interaction between an electronic device 100 and an electronic device 200 when the electronic device 100 is activated when a user wears the electronic device 100 according to an embodiment of this application;
FIG. 12 is a diagram of interaction between an electronic device 100 and an electronic device 200 when the electronic device 100 is activated before a user wears the electronic device 100 according to an embodiment of this application;
FIG. 13 is a diagram of an overall structure of an electronic device 100 according to an embodiment of this application;
FIG. 14 is a diagram of a hardware structure of an electronic device 200 according to an embodiment of this application; and
FIG. 15 is a block diagram of a software structure of an electronic device 200 according to an embodiment of this application.

### DESCRIPTION OF EMBODIMENTS

The technical solutions in embodiments of this application are clearly described in detail below with reference to the accompanying drawings. In the descriptions of embodiments of this application, unless otherwise stated, "/" represents the meaning of "or". For example, A/B may represent A or B. "And/or" in the text merely describes an association relationship between associated objects and represents that three relationships may exist. For example, A and/or B may represent the following three cases: Only A exists, both A and B exist, and only B exists. In addition, in the descriptions of embodiments of this application, "a plurality of" means two or more than two.

Hereinafter, the terms "first" and "second" are used only for descriptive purposes, and cannot be understood as indicating or implying relative importance or implicitly indicating a quantity of indicated technical features. Therefore, a feature limited by "first" and "second" may explicitly or implicitly include one or more features. In the descriptions of embodiments of this application, unless otherwise stated, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language such as Java or an extensible markup language (extensible markup language, XML). Interface source code is parsed and rendered on an electronic device, and is finally presented as content that can be recognized by the user. The user interface is usually represented in a form of a graphical user interface (graphic user interface, GUI), and is a user interface that is related to a computer operation and that is displayed in a graphic manner. The user interface may be a visual interface element such as a text, an icon, a button, a menu, a tab, a text box, a dialog box, a status bar, a navigation bar, or a Widget that is displayed on a display of the electronic device.

For some devices that need to be worn on a user, have a low operating current, and have a low battery capacity, for example, a CGM device, because there is a long inventory time from shipment and sale to activation and use after the user purchases the device, if the device remains in a standby state within the inventory time, battery power of the device is continuously consumed. Taking one-year inventory time as an example, a standby power current of 100 nA corresponds to roughly 1 mAh of battery capacity loss.

Therefore, to reduce battery power consumption during the period from shipment to use of the device by the user, the device may be in an inactive state in the inventory phase, that is, no power is supplied to the device. When the user needs to use the device, the user manually activates the device. For example, for the CGM, common manners are as follows:
(1) A mechanical switch is added to a power path of the CGM device. Before using the CGM device, the user first manually or automatically close the switch to supply power to the CGM device, thereby activating the device.
(2) A charging circuit is disposed in the CGM device. Before using the CGM device, the user charges a battery by using the charging circuit, to activate the device .
(3) A battery and a mainboard of the CGM device are prepared into two independent components. Before using the CGM device, the user first mounts the two components together to activate the device.

Although the foregoing three manners can reduce or avoid battery power consumption of the CGM device in the inventory period to some extent, the mechanical switch used in the manner 1 is large in size and difficult to be integrated, and the user needs to manually close the switch before using the device. In the manner 2, the user needs to charge the CGM device. In the manner 3, the components need to be manually mounted by the user before the user uses the device. Operations are not convenient and seamless activation cannot be implemented. In addition, in the manner 1, the switch may be accidentally touched due to external force in a wearing process of the user, making the device disabled, and affecting user experience. In the manner 2, a charger further needs to be provided by a manufacturer, and costs increase.

Therefore, how to implement seamless activation of the device and not to trigger the device intentionally or accidentally during use is a problem that needs to be urgently resolved currently.

An embodiment of this application provides a device activation circuit. The device activation circuit is connected to a battery, a power management module, and a data processing module in an electronic device 100. The power management module may be configured to: receive an input from the battery, and supply power to the data processing module. The data processing module may be configured to provide a corresponding computing capability for work of the electronic device 100, for example, determine a body indicator of a user based on obtained data. The activation circuit may be configured to detect whether the user wears the electronic device 100 or whether the user prepares to wear the electronic device 100. When detecting that the user wears the electronic device 100 or the user prepares to wear the electronic device 100, the activation circuit may switch a circuit between the power management module and the battery from a cut-off state to a conducted state, so that the battery can supply power to the data processing module by using the power management module, thereby activating the device.

The electronic device 100 may be a device that needs to be worn on the user, has a low operating current, and has a low battery capacity, for example, a CGM device, a CKM device, a CLM device, or an electrocardiogram patch. The user may monitor the body indicator of the user by wearing the electronic device 100. The body indicator may include but is not limited to blood glucose, a heart rate, a body temperature, blood pressure, blood lipid, and blood ketone. Before being used by the user, this type of device needs to go through a period of inventory phase. By using the device activation circuit provided in this embodiment of this application, the electronic device 100 can be prevented from being still in an active state in the inventory phase, so that power consumption of the electronic device 100 in the inventory phase is reduced, and a battery use time of the electronic device 100 is prolonged.

The device activation circuit may include a switching module and a sensing module. The sensing module may be configured to detect whether the user wears the electronic device 100 or whether the user prepares to wear the electronic device 100. The switching module may be configured to control cut-off and conducted states of the circuit between the power management module and the battery.

The switching module may include one or more metal oxide semiconductor (Metal Oxide Semiconductor, MOS) field-effect transistors. The one or more MOS transistors may change between conducted and cut-off states of the one or more MOS transistors based on voltage changes in the circuit, to implement connection and disconnection of the circuit between the power management module and the battery.

The sensing module may include a sensor, for example, an optical sensor, a magnetic sensor, or a temperature sensor. These sensors may determine, by sensing changes in surrounding environment, whether the user wears the electronic device 100 or whether the user prepares to wear the electronic device 100. For a specific sensing principle of the sensor, refer to subsequent content. Details are not described herein.

It can be learned that, based on the device activation circuit provided in this embodiment of this application, before the user uses the electronic device 100, the circuit between the power management module and the battery of the electronic device 100 can be isolated, to effectively reduce battery loss of the electronic device 100 in the inventory phase. In addition, when the user prepares to wear the device or has worn the device, the device activation circuit can automatically conduct the circuit between the power management module and the battery, so that the battery supplies power to the data processing module by using the power management module, to activate the electronic device 100, and enable the electronic device 100 to enter a working state. The electronic device 100 does not need to be equipped with a switch, or work such as charging or assembly does not need to be performed on the electronic device 100 before use, to facilitate an operation of the user, thereby implementing seamless activation of the device when the user wears the electronic device 100, and preventing intentional or accidental triggering during use.

**To better understand an activation principle of the device activation circuit, the following uses an example in which the electronic device 100 is a CGM device to describe an operation manner when the user wears the electronic device 100.**

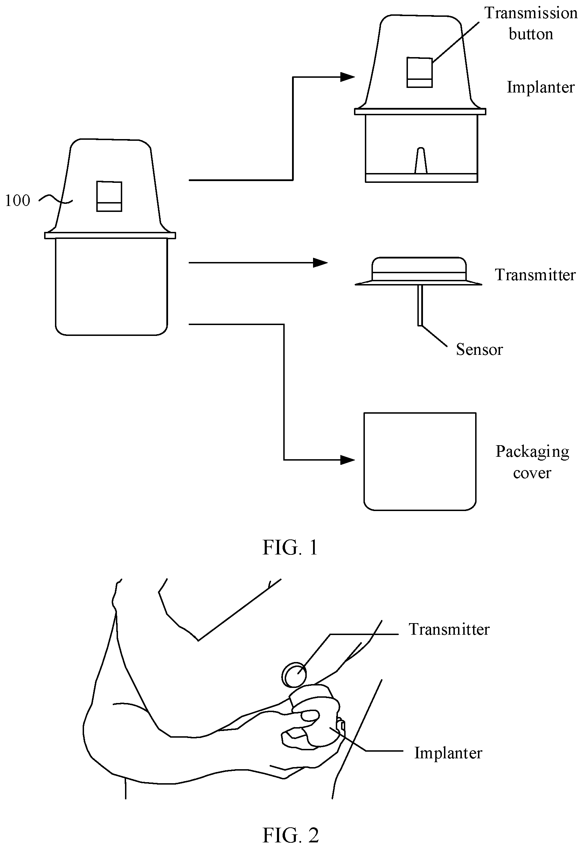

**FIG. 1** **is a diagram of a structure of an electronic device 100 according to an embodiment of this application.**

The electronic device 100 may be a CGM device, where the device may be configured to collect a blood glucose value of a user in real time. A specific implementation principle is as follows: The electronic device 100 may implant a sensor (an electrochemical sensor) under the user's skin to be in contact with interstitial fluid, to determine glucose concentration in the interstitial fluid, and then determine a blood glucose value of the user based on the glucose concentration in the interstitial fluid.

As shown in FIG. 1, the electronic device 100 may include three parts: an implanter, a transmitter, and a packaging cover. Before use, the transmitter is located in the implanter, the implanter includes a transmission button, and the user may detach the transmitter from the implanter by acting on the transmission button. The transmitter is equipped with a sensor. After the sensor is implanted under the skin, the electronic device 100 may calculate the blood glucose value of the user based on data collected by the sensor.

**FIG. 2** **is a diagram of an operation of using an electronic device 100.**

As shown in FIG. 2, when the user uses the electronic device 100, the user may first remove the packaging cover, then align and attach the implanter to a position where the user needs to wear the transmitter, for example, an upper arm or a belly, and then press or push the transmission button to push out the transmitter in the implanter, so that the transmitter is attached to skin, and the sensor is implanted under the skin at the same time. Then, a receiving device such as dedicated reader/writer, a mobile phone, or a watch may be used, so that the blood glucose value collected by the transmitter is transmitted to the receiving device, and the receiving device displays or broadcasts the blood glucose value.

It may be understood that, if the electronic device 100 is another device, for example, a CKM device, a CLM device, or an electrocardiogram patch, these devices have a similar structure as the CGM device, and an operation manner of wearing the devices by the user is also similar. Details are not described herein again.

It should be noted that, the core idea of this application is to determine, based on an environmental change sensed by the sensor, a status of using the device by the user, so as to determine whether to activate the device. The structure of the electronic device 100 shown in FIG. 1 and the diagram of the operation of using the electronic device 100 shown in FIG. 2 are merely examples, and do not constitute a limitation on this application. The electronic device 100 may further have another structure, for example, include only the transmitter and the implanter, or include only the transmitter and the packaging cover. In addition, names of the transmitter, the implanter, and the packaging cover do not constitute a limitation on the structure of the electronic device 100. It should be understood that the transmitter is a component that is finally worn on the user to measure an indicator of the user, the implanter is configured to assist the user in wearing the transmitter, the packaging cover plays a role of protecting a product and facilitating storage and transportation, and any other component that has a same function should fall within the protection scope of this application. For example, in another embodiment of this application, the packaging cover may alternatively be replaced with a packaging box. This is not limited in this embodiment of this application.

In this embodiment of this application, the transmitter may also be referred to as a first component, the implanter may also be referred to as a second component, and the packaging cover may also be referred to as a third component.

If the electronic device 100 is the CGM device shown in FIG. 1 and FIG. 2, the device activation circuit provided in this embodiment of this application may be located in the transmitter of the electronic device 100. The device activation circuit may be configured to power on a circuit inside the transmitter when the transmitter is implanted under the skin of the user or before the transmitter is implanted under the skin of the user, so that after being implanted under the skin, the transmitter can calculate the blood glucose value of the user based on data collected by the sensor implanted under the skin.

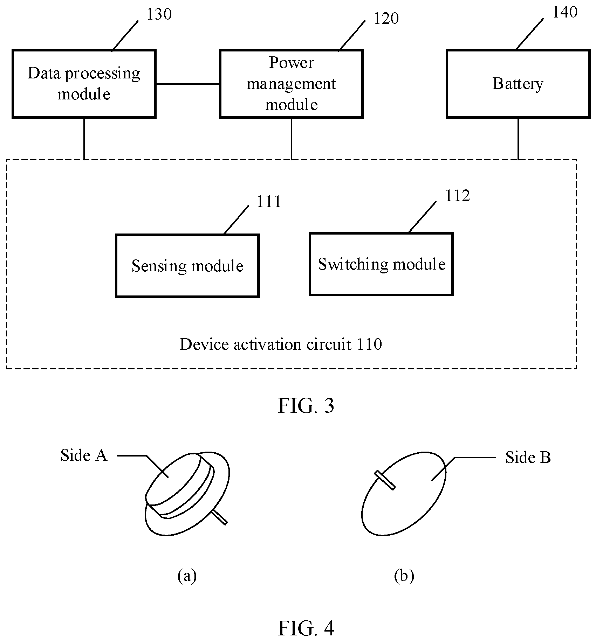

**FIG. 3** **is a diagram of a structure of a device activation circuit 110 according to an embodiment of this application.**

As shown in FIG. 3, the device activation circuit 110 may include a sensing module 111 and a switching module 112.

The sensing module 111 may include a sensor, for example, an optical sensor, a magnetic sensor, or a temperature sensor. These sensors may reflect, by sensing changes in surrounding environment, whether the electronic device 100 (or the transmitter) is worn on the user or whether the user is in a preparation phase of wearing the device, to further change a voltage in a circuit.

The switching module 112 includes one or more MOS transistors. The one or more MOS transistors may change between conducted and cut-off states of the one or more MOS transistors based on the voltage changes in the circuit, to further change a connection status of the circuit.

In addition, the device activation circuit 110 is further connected to a power management module 120, a data processing module 130, and a battery 140 in the electronic device 100. The power management module 120 is connected to the data processing module 130. The power management module 120 may be configured to receive an input of the battery 140, and supply power to the data processing module 130. For example, the power management module 120 may include a power management unit (power management unit, PMU). The data processing module 130 may be configured to provide a corresponding computing capability for work of the electronic device 100. For example, if the electronic device 100 is a CGM device, the data processing module 130 may be configured to provide a corresponding computing capability for the electronic device 100 to measure a blood glucose value. For example, the data processing module 130 may include a microcontroller unit (microcontroller unit, MCU).

In a working process of the device activation circuit 110, the one or more MOS transistors in the switching module 112 may change between conducted and cut-off states of the one or more MOS transistors based on the voltage changes in the circuit, to implement connection and disconnection of the circuit between the power management module 120 and the battery 140. If the circuit between the power management module 120 and the battery 140 is disconnected, the battery 140 cannot supply power to the data processing module 130 by using the power management module 120, and the power management module 120 does not consume power. In this case, the electronic device 100 is in an inactive state. If the circuit between the power management module 120 and the battery 140 is connected, the battery 1409 may supply power to the data processing module 130 by using the power management module 120, and the data processing module 130 may provide a corresponding computing capability for work of the electronic device 100 in a power-on condition. In this case, the electronic device 100 is in an active state.

In some implementations, the one or more MOS transistors in the switching module 112 may be integrated into a chip, so that an area occupied by the device activation circuit 110 and power loss can be further reduced.

In a specific implementation, when the user uses the electronic device 100, a series of operations such as removing the packaging cover and wearing the device exist. Therefore, in a process in which the user wears the device, the sensing module 111 may sense changes in environment, to further change the voltage in the device activation circuit 110. Then, the switching module 112 switches a disconnected state of the circuit between the power management module 120 and the battery 140 to a connected state based on the changed voltage, so that the battery 140 supplies power to the data processing module 130 by using the power management module 120, to enable the data processing module 130 to start to work, thereby completing activation of the device.

Further, after the data processing module 130 starts to work, the data processing module 130 may further reversely control conduction and cut-off of the one or more MOS transistors in the switching module 112, and isolate impact of the voltage change of a loop in which the sensing module 111 is located on the switching module 112, to maintain a state in which the power management module 120 supplies power to the data processing module 130. In this way, after the device is activated, changes in environment surrounding the sensing module 111 can be prevented from affecting power supply to the data processing module 130 by the power management module 120. In other words, after the device is activated, the electronic device can still remain an active state, and is not affected by factors such as surrounding light, magnetic field, or temperature, to ensure normal use of the electronic device 100 by the user after the device is activated.

**Because the sensor in the sensing module 111 may have a plurality of different types, the following describes in detail a working principle of the device activation circuit 110 by using an optical sensor and a magnetic sensor as an example.**

### (1) Optical sensor

Because the optical sensor may sense changes in illumination and change a voltage in a circuit, based on different positions at which the optical sensor is placed in the electronic device 100, the optical sensor may sense changes in illumination in different phases of using the electronic device 100 by the user.

FIG. 4 is a multi-angle diagram of a transmitter in the electronic device 100 by using an example in which the electronic device 100 is a CGM device.

With reference to subcutaneous implantation of the transmitter, (a) in FIG. 4 shows a side (side A) of the transmitter facing away from the skin of the user, and (b) in FIG. 4 shows a side (side B) of the transmitter facing or adhered to the skin of the user.

In some implementations, the optical sensor may be disposed on the side A of the transmitter. Before the transmitter is implanted under the skin of the user, the transmitter is located in the implanter, the side A faces the implanter, and no light is illuminated onto the side A. Therefore, only when the transmitter is implanted under the skin of the user, that is, the user wears the electronic device 100, the side A of the transmitter is exposed to illumination and senses changes in illumination, to change a voltage of the device activation circuit 110, so that the device activation circuit 110 activates the electronic device 100.

In some other implementations, the optical sensor may be disposed on the side B of the transmitter. In this case, the packaging cover of the electronic device 100 may be made of an opaque material. In this way, provided that the user does not remove the packaging cover on the electronic device 100, the side B of the transmitter is in an opaque environment. If the user removes the packaging cover of the electronic device 100, light can be illuminated onto the side B of the transmitter through a side on which the transmitter is placed in the implanter. Therefore, provided that the user removes the packaging cover of the electronic device 100, that is, the user prepares to implant the transmitter under the skin of the user, that is, in a preparation phase of wearing the device by the user, the side B of the transmitter is exposed to illumination, and senses changes in illumination, to change the voltage in the device activation circuit 110, so that the device activation circuit 110 activates the electronic device 100.

For example, that the packaging cover is opaque may mean that intensity of light that can be transmitted through the packaging cover is less than 0.0001 Lux.

It may be understood that the optical sensor may alternatively be disposed at another position of the transmitter, and the position enables the optical sensor to sense changes in illumination when the user wears or prepares to wear the device. This is not limited in this embodiment of this application.

The following uses a specific circuit structure to describe a working principle of the device activation circuit 110 when the sensing module 111 includes an optical sensor.

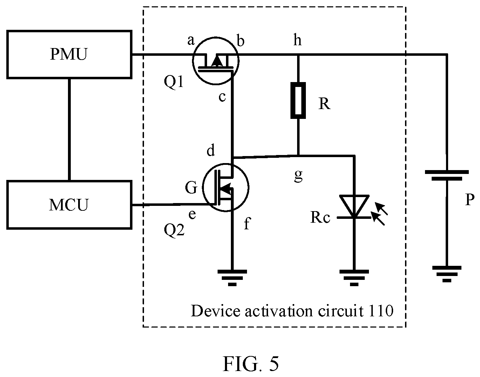

**FIG. 5** **is a diagram of a circuit structure of a device activation circuit 110 according to an embodiment of this application.**

As shown in FIG. 5, the device activation circuit 110 may include components such as a power supply P, a photodiode Rc, a resistor R, a PMOS transistor Q1, and an NMOS transistor Q2.

A drain (with reference to a point a) of Q1 is connected to one end of the PMU, a source (with reference to a point b) of Q1 is connected to one end of P, a gate (with reference to a point c) of Q1 is connected to one end (with reference to a point g) of Rc, the other end of Rc is grounded, the other end of P is grounded, and the other end of the PMU is connected to one end of the MCU.

Further, one end (with reference to a point h) of R is connected to the source of Q1, and the other end (with reference to the point g) of R is connected to the gate of Q1.

It should be understood that the resistor R is an optional component, and the resistor R can reduce a leakage current in the device activation circuit 110 before the electronic device 100 is activated.

Further, a drain (with reference to a point d) of Q2 is connected to the gate of Q1, a gate (with reference to a point e) of Q2 is connected to the other end of the MCU, and a source (with reference to a point f) of Q2 is grounded.

Rc may be an optical sensor in the sensing module, and Rc may sense changes in surrounding light. Specifically, Rc may be in a cut-off state when not sensing light, and may be in a conducted state when sensing light.

Q1 and Q2 may be MOS transistors included in the switching module, and these MOS transistors may change between conducted and cut-off states of the MOS transistors based on voltage changes of pins of the MOS transistors. The PMOS transistor is conducted when a gate-source voltage Vgs is less than a threshold voltage Vth, and is cut off when Vgs is greater than Vth. The NMOS transistor is conducted when Vgs is greater than Vth, and is cut off when Vgs is less than Vth.

It may be understood that the device activation circuit 110 may further include another component, for example, a resistor or a capacitor. The circuit structure shown in FIG. 5 does not constitute a limitation on this embodiment of this application.

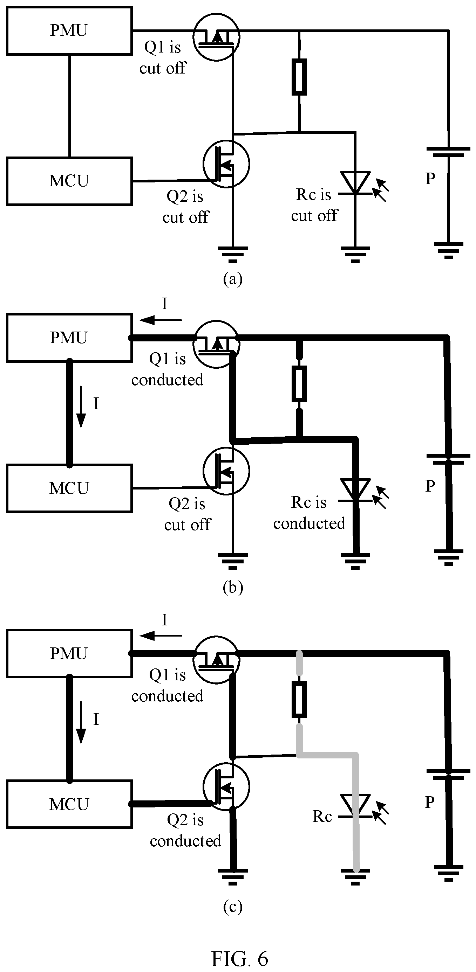

With reference to FIG. 6, the following describes a diagram of a working principle of the device activation circuit 110 shown in FIG. 5 by using three states.

### (1) No-illumination state

As shown in (a) in FIG. 6, the no-illumination state may be a state in which Rc does not sense illumination. In this case, Rc is in a cut-off state, Q1 is in a cut-off state, and Q2 is in a cut-off state. Therefore, in the no-illumination state, the power supply P cannot supply power to the MCU by using the PMU, and the electronic device 100 is in an inactive state.

### (2) State of entering illumination

As shown in (b) in FIG. 6, the state of entering illumination means that Rc changes from not sensing illumination to a state of sensing illumination. In this case, Rc switches to the conducted state due to impact of illumination. In addition, conduction of Rc enables Q1 to switch to the conducted state, and Q2 continues to be in the cut-off state. In this case, under effect of illumination, the power supply P may supply power to the MCU by using the PMU, and the electronic device 100 is activated.

For example, when sensing that light intensity is greater than a first threshold (for example, 0.01 Lux), Rc may switch from being cut off to being conducted. It may be understood that the first threshold is related to the Rc component, and the first threshold is not limited in this embodiment of this application.

In this state, for a conducted line in the circuit, refer to a bold line segment shown in (b) in FIG. 6.

### (3) Stable state

As shown in (c) in FIG. 6, after the MCU is powered on, the MCU may output a high level by using a pin connected to the Q2, so that Q2 switches to the conducted state, thereby controlling the Q1 to be continuously in the conducted state. In this case, regardless of whether Rc senses light, a state of a loop (with reference to a gray line segment shown in (b) in FIG. 6) of Rc does not affect the state of Q1. In this way, the electronic device 100 can be stably in an active state regardless of whether the electronic device 100 is in an illumination environment.

In this state, for a conducted line in the circuit, refer to a bold line segment shown in (c) in FIG. 6.

It can be learned from FIG. 6 that the foregoing three states are successively experienced from a time when the user unpacks the electronic device 100 to a time when the user wears the electronic device 100, so that the electronic device 100 can be automatically activated when the user prepares to wear the electronic device 100 or wears the electronic device 100, thereby reducing troubles of manual activation by the user.

### (2) Magnetic sensor

Because the magnetic sensor may sense changes in a magnetic field and change voltage in a circuit, a magnet may be placed in the implanter or the packaging cover. In this way, the magnetic sensor may sense the changes in the magnetic field in different phases of using the electronic device 100 by the user.

For example, the electronic device 100 is the CGM device shown in FIG. 1 or FIG. 2.

In some implementations, the magnet may be placed in the implanter. When the transmitter is implanted under the skin of the user, the transmitter is detached from the implanter. As a result, the magnetic sensor is far away from magnetic field effect of the magnet. In this way, provided that the transmitter is implanted under the skin of the user, that is, the user wears the electronic device 100, the magnetic sensor can sense the changes in the magnetic field, to change the voltage in the device activation circuit 110, so that the device activation circuit 110 activates the electronic device 100.

In some other implementations, the magnet may be placed in the packaging cover. When the user does not remove the packaging cover of the electronic device 100, a position of the magnet relative to the transmitter does not change, and the magnetic sensor is always under the magnetic field effect of the magnet. If the user removes the packaging cover of the electronic device 100, the magnetic sensor is far away from the magnetic field effect of the magnet. In this way, provided that the user removes the packaging cover of the electronic device 100, that is, the user prepares to implant the transmitter under the skin of the user, the magnetic sensor can sense the changes in the magnetic field, to change the voltage in the device activation circuit 110, so that the device activation circuit 110 activates the electronic device 100.

It may be understood that the magnet may be alternatively disposed at another position. This is not limited in this embodiment of this application.

The following uses a specific circuit structure to describe a working principle of the device activation circuit 110 when the sensing module 111 includes a magnetic sensor.

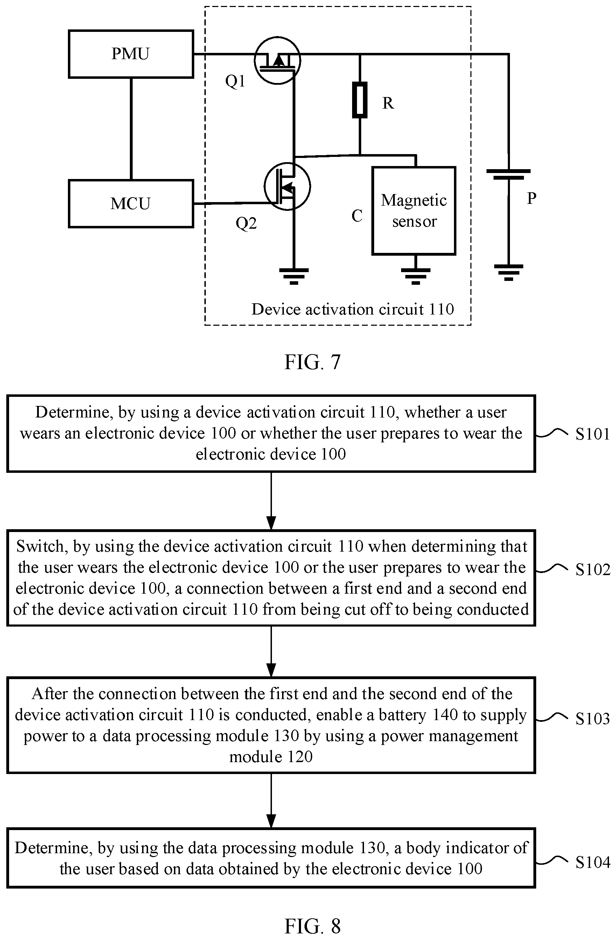

**FIG. 7** **is a diagram of a circuit structure of another device activation circuit 110 according to an embodiment of this application.**

As shown in FIG. 7, the device activation circuit 110 may include components such as a power supply P, a magnetic sensor C, a resistor R, a PMOS transistor Q1, and an NMOS transistor Q2.

It may be understood that the diagram of the circuit structure shown in FIG. 7 is similar to the diagram of the circuit structure shown in FIG. 5. A difference lies in that, in the diagram of the circuit structure shown in FIG. 7, the photodiode Rc is replaced with the magnetic sensor C based on the circuit diagram shown in FIG. 5. For specific structural descriptions of the diagram of the circuit structure shown in FIG. 7, refer to the structural descriptions of the diagram of the circuit structure in FIG. 5. Details are not described herein again.

The magnetic sensor C may include any one of the following: an anisotropic magnetoresistance (Anisotropic Magnetoresistance, AMR) sensor, a tunnel magnetoresistance (Tunnel Magnetoresistance, TMR) sensor, a giant magnetoresistance (Giant Magnetoresistance, GMR) sensor, a magnetic reed switch, a Hall switch, or the like.

The following describes a working principle of the device activation circuit 110 shown in FIG. 7 by using three states.

### (1) Magnetic effect state

The magnetic effect state is a state in which the magnetic sensor C is under magnetic field effect of the magnet. In this case, the magnetic sensor C is in a cut-off state due to the magnetic field effect, so that Q1 is in the cut-off state, and Q2 is in the cut-off state. Therefore, in the magnetic effect state, the power supply P cannot supply power to the MCU by using the PMU, and the electronic device 100 is in an inactive state.

### (2) State of being far away from magnetic effect

The state of being far away from magnetic effect is a state in which the magnetic sensor C is far away from the magnetic field effect. In this case, the magnetic sensor C switches to the conducted state because the magnetic sensor C gets out of the magnetic field effect. In addition, conduction of the magnetic sensor C enables Q1 to switch to the conducted state, and Q2 continues to be in the cut-off state. In this case, in the state of being far away from the magnetic effect, the power supply P may supply power to the MCU by using the PMU, and the electronic device 100 is activated.

For example, the magnetic sensor C may switch from being cut off to being conducted when sensing that magnetic field strength is less than a second threshold. It may be understood that the second threshold is related to the magnetic sensor C component. The second threshold is not limited in this embodiment of this application.

### (3) Stable state

After the MCU is powered on, the MCU may input a high level by using a pin connected to the Q2, so that Q2 switches to the conducted state, thereby controlling the Q1 to be continuously in the conducted state. In this case, regardless of whether the magnetic sensor C returns to the magnetic field effect, the state of the loop of the magnetic sensor C does not affect the state of Q1. In this way, the electronic device 100 can be stably in an active state regardless of whether the electronic device 100 is under the magnetic field effect.

It can be learned that the foregoing three states are successively experienced from a time when the user unpacks the electronic device 100 to a time when the user wears the electronic device 100, so that the electronic device 100 can be automatically activated when the user prepares to wear the electronic device 100 or wears the electronic device 100, thereby reducing troubles of manual activation by the user.

It may be understood that the working principle of the device activation circuit 110 shown in FIG. 7 is similar to the working principle of the device activation circuit 110 shown in FIG. 5. For specific content that is not described in detail in the working principle of the device activation circuit 110 shown in FIG. 7, refer to the related content in FIG. 5 and FIG. 6. Details are not described herein again.

It should be noted that the circuit structures shown in FIG. 5 to FIG. 7 are merely examples, and another circuit structure or sensor may be used, to implement device activation based on an environmental change of a device. For example, in another embodiment of this application, the electronic device 100 may alternatively be activated by detecting that the illuminance meets a specific condition, for example, changes from high to low, or the magnetic field strength meets a specific condition, for example, changes from weak to strong. It should be understood that a solution of determining, based on an environment condition of the device, whether to activate the device should fall within the protection scope of this application.

**FIG. 8** **is a schematic flowchart of a device activation method according to an embodiment of this application.**

As shown in FIG. 8, the device activation method may include the following steps.

**S101:** An electronic device 100 determines, by using a device activation circuit 110, whether a user wears the electronic device 100 or whether a user prepares to wear the electronic device 100.

The electronic device 100 is configured to measure a body indicator of the user. The electronic device 100 may be a device that needs to be worn on the user, is configured to measure the body indicator of the user, has a low operating current, and has a low battery capacity.

For example, if the electronic device 100 is a CGM device, the body indicator may be blood glucose; if the electronic device 100 is a CKM device, the body indicator may be blood ketone; if the electronic device 100 is a CLM device, the body indicator may be lactate; or if the electronic device 100 is an electrocardiogram patch, the body indicator may be an electrocardiogram signal.

The electronic device 100 may include modules such as the device activation circuit 110, a power management module 120, a data processing module 130, and a battery 140.

The device activation circuit 110 may be configured to activate the electronic device 100, that is, the battery 140 can supply power to the data processing module 130 by using the power management module 120. The device activation circuit 110 may include a sensing module 111 and a switching module 112. The sensing module 111 is connected to the switching module 112.

The sensing module 111 may sense changes in surrounding environment of the electronic device 100, and control voltage changes surrounding the sensing module 111 in a changing environment. The changes in the environment may include changes of factors such as light, temperature, and magnetic field. For example, if the changes in the environment includes the changes in light, the sensing module 111 may include an optical sensor. For another example, if the changes in the environment includes the changes in magnetic field, the sensing module 111 may include a magnetic sensor.

In this embodiment of this application, a sensor located in the device activation circuit 110 may also be referred to as a first sensor.

The switching module 112 may control connection and disconnection of a line under voltage changes surrounding the sensing module 111.

The switching module 112 may disconnect a line between the battery 140 and the power management module 120, so that the battery 140 cannot supply power to the data processing module 130 by using the battery management module 120. The switching module 112 may further connect the line between the battery 140 and the power management module 120, so that the battery 140 can supply power to the data processing module 130 by using the battery management module 120.

It should be noted that before the surrounding environment of the electronic device 100 changes, the switching module 112 keeps the line between the battery 140 and the power management module 120 in a disconnected state, so that the battery 140 cannot supply power to the data processing module 130 by using the battery management module 120. In this case, the electronic device 100 is in an inactive state.

The power management module 120 may be configured to receive an input of the battery 140, and supply power to each module in the electronic device 100, for example, the data processing module 130.

The data processing module 130 may be configured to provide a computing capability for the electronic device 100. For example, the data processing module 130 may be configured to determine the body indicator of the user based on data obtained by the electronic device 100. For example, if the electronic device 100 is a CGM device, the electronic device 100 may be configured to determine blood glucose of the user based on data collected by a sensor in the CGM device, for example, a current value.

For specific descriptions of the device activation circuit 110, the power management module 120, the data processing module 130, and the battery 140, refer to the related content in FIG. 3.

The device activation circuit 110 may include a first end and a second end, where the first end is connected to the power management module 120, the second end is connected to the battery 140, and the power management module 120 is connected to the data processing module 130.

For example, with reference to FIG. 5, the first end may be the point a, and the second end may be the point b.

Specifically, the device activation circuit 110 may include a first MOS transistor and a first sensor. A drain of the first MOS transistor is the first end of the device activation circuit 110, a source of the first MOS transistor is the second end of the device activation circuit 110, one end of the first sensor is connected to a gate of the first MOS transistor, and the other end of the first sensor is grounded.

The first sensor may be a component such as an optical sensor, a magnetic sensor, or a temperature sensor.

For example, with reference to FIG. 5, the first MOS transistor may be the PMOS transistor Q1, and the first sensor may be the photodiode Rc. For another example, with reference to FIG. 7, the first MOS transistor may be the PMOS transistor Q1, and the first sensor may be the magnetic sensor C.

That the electronic device 100 detects, by using the device activation circuit 110, whether the user wears the electronic device 100 or whether the user prepares to wear the electronic device 100 may specifically include: The electronic device 100 detects, by using the first sensor, whether the user wears the electronic device 100 or whether the user prepares to wear the electronic device 100.

When detecting that the user wears the electronic device 100 or the user prepares to wear the electronic device 100, the first sensor may switch from being cut off to being conducted. Further, when the first sensor is cut off, the first MOS transistor is cut off, and when the first sensor is conducted, the first MOS transistor is conducted.

It may be understood that, for the circuit principle in step S101, refer to the foregoing part of the working principle of the circuit in the no-illumination state and the magnetic effect state mentioned in FIG. 5 to FIG. 7.

**S102:** The electronic device 100 switches, by using the device activation circuit 110 when determining that the user wears the electronic device 100 or the user prepares to wear the electronic device 100, a connection between the first end and the second end of the device activation circuit 110 from being cut off to being conducted.

When the electronic device 100 detects that the user wears the electronic device 100 or the user prepares to wear the electronic device 100, the first sensor in the device activation circuit 110 switches from being cut off to being conducted, so that the first MOS transistor in the device activation circuit 110 switches from being cut off to being conducted. In this way, the connection between the first end and the second end of the device activation circuit 110 is switched from being cut off to being conducted.

The device activation circuit 110, the power management module 120, the data processing module 130, and the battery 140 may all be located in a transmitter of the electronic device 100.

If the first sensor is an optical sensor, the following two cases may exist:
(1) The transmitter in the electronic device 100 may be located in an implanter. When the user wears the electronic device 100, the transmitter is detached from the implanter. The first sensor is disposed on a side facing the implanter when the transmitter is located in the implanter.

For example, the side facing the implanter may be the side A shown in FIG. 4.

In this case, that the electronic device 100 detects, by using the first sensor, whether the user wears the electronic device 100 may specifically include: The electronic device 100 detects, based on an illumination change sensed by the first sensor, whether the user wears the electronic device 100.

The first sensor switches from being cut off to being conducted when sensing that light intensity is greater than a first threshold.

Specifically, the first sensor may sense that the light intensity is greater than the first threshold when the transmitter is detached from the implanter.

(2) The transmitter in the electronic device 100 is covered by a packaging cover, and the packaging cover is opaque. When the user prepares to wear the electronic device 100, the packaging cover is removed, and the first sensor is disposed on a side facing the packaging cover.

For example, the side facing the packaging cover may be the side B shown in FIG. 4.

In this case, that the electronic device 100 detects, by using the first sensor, whether the user prepares to wear the electronic device 100 may specifically include: The electronic device 100 detects, by using an illumination change sensed by the first sensor, whether the user prepares to wear the electronic device 100.

The first sensor switches from being cut off to being conducted when sensing that light intensity is greater than a first threshold.

Specifically, the first sensor may sense that the light intensity is greater than the first threshold when the user removes the packaging cover.

If the first sensor is a magnetic sensor, the following two cases may exist:
(1) The transmitter in the electronic device 100 may be located in an implanter. When the user wears the electronic device 100, the transmitter is detached from the implanter. The implanter may include a magnet.

In this case, that the electronic device 100 detects, by using the first sensor, whether the user wears the electronic device 100 may specifically include: The electronic device 100 detects, based on changes in a magnetic field sensed by the first sensor, whether the user wears the electronic device 100.

The first sensor switches from being cut off to being conducted when sensing that magnetic field strength of the magnet is less than a second threshold.

Specifically, the first sensor may sense that the magnetic field strength of the magnet is less than the second threshold when the transmitter is detached from the implanter.

(2) The transmitter in the electronic device 100 is covered by the packaging cover, and the packaging cover is removed when the user prepares to wear the electronic device 100. The packaging cover includes a magnet.

In this case, that the electronic device 100 detects, by using the first sensor, whether the user prepares to wear the electronic device 100 may specifically include: The electronic device 100 detects, by using changes in a magnetic field sensed by the first sensor, whether the user prepares to wear the electronic device 100.

The first sensor switches from being cut off to being conducted when sensing that magnetic field strength of the magnet is less than a second threshold.

Specifically, the first sensor may sense that the magnetic field strength of the magnet is less than the second threshold when the user removes the packaging cover.

It may be understood that, for the circuit principle in step S102, refer to the foregoing part of the working principle of the circuit in the state of entering illumination and the state of being far away from magnetic effect mentioned in FIG. 5 to FIG. 7.

In some implementations, the device activation circuit 110 may further include a first resistor, where one end of the first resistor is connected to the gate of the first MOS transistor, and the other end is connected to the gate of the first MOS transistor. The first resistor may be configured to reduce a leakage current in the device activation circuit 110.

In some implementations, the magnetic sensor is any one of an AMR sensor, a TMR sensor, a GMR sensor, a magnetic reed switch, or a Hall switch.

In some implementations, the power management module may include a PMU, and the data processing module may include any one of an MCU, a digital signal processor, a advanced RISC machine (advanced RISC machine, ARM) processor, a field programmable gate array (field programmable gate array, FPGA), or an application-specific integrated circuit (application-specific integrated circuit, ASIC).

**S103:** After the connection between the first end and the second end of the device activation circuit 110 is conducted, the electronic device 100 enables the battery 140 to supply power to the data processing module 130 by using the power management module 120.

After the battery 140 supplies power to the data processing module 130 by using the power management module 120, the data processing module 130 is in a power-on state, to implement activation of the electronic device 100.

After the electronic device 100 is activated, the surrounding environment may change again. Therefore, to prevent the sensing module 111 from affecting the activated state of the electronic device 100 due to the changes in the surrounding environment, the electronic device 100 may output a voltage by using the data processing module 130 after the data processing module 130 is powered on, and reversely control the voltage surrounding the switching module 112, so that the switching module 112 is no longer affected by the voltage surrounding the sensing module 111, thereby ensuring that the power supply 140 can continuously supply power to the data processing module 130 in the electronic device 100.

Further, when the data processing module 130 is powered on, the electronic device 100 may control the connection between the first end and the second end of the device activation circuit 110 to be continuously conducted.

In a specific implementation, the device activation circuit 110 may further include a second MOS transistor. A gate of the second MOS transistor is connected to the data processing module 130, a source of the second MOS transistor is grounded, and a drain of the second MOS transistor is connected to the gate of the first MOS transistor.

For example, with reference to FIG. 5 or FIG. 7, the second MOS transistor may be an NMOS transistor Q2.

After the electronic device 100 enables the battery 140 to supply power to the data processing module 130 by using the power management module 120, the electronic device 100 may output a first voltage to the gate of the second MOS transistor by using the data processing module 130 when the data processing module 130 is powered on. Then, the electronic device 100 may enable the first MOS transistor to be continuously conducted by using the second MOS transistor when the gate of the second MOS transistor receives the first voltage, where the second MOS transistor switches from being cut off to being conducted when the gate of the second MOS transistor receives a high voltage.

The first voltage is greater than a voltage originally output by the data processing module to the gate of the second MOS transistor. Further, optionally, the first voltage is the high voltage greater than a threshold. It should be understood that the voltage originally output by the data processing module to the gate of the second MOS transistor may be 0.

It may be understood that, for the circuit principle in step S103, refer to the foregoing part of the working principle of the circuit entering the stable state mentioned in FIG. 5 to FIG. 7.

**S104:** The electronic device 100 determines, by using the data processing module 130, the body indicator of the user based on the data obtained by the electronic device 100.

After the electronic device 100 is activated, the electronic device 100 may start to obtain data, for example, collect data by using a second sensor, and process the data by using the data processing module 130, to determine the body indicator of the user, thereby measuring the body indicator of the user.

In conclusion, based on the device activation method provided in this embodiment of this application, a specific operation manner used when the user wears the device may be used to apply a sensing characteristic of the sensor on the surrounding environment, to avoid a trouble of manual activation when the user wears the device and implement seamless activation of the electronic device by the user, thereby improving experience of the user in using this type of wearable device.

After the device is activated, the electronic device 100 may start to work, measure the body indicator of the user, and send the data collected by the electronic device 100 to another device for display or broadcasting.

For example, the electronic device 100 is a CGM device. The electronic device 100 may search for a surrounding device (referred to as an electronic device 200 below) that may be configured to output a blood glucose value, and establish a communication connection to the electronic device 200. In this way, the electronic device 100 may send a collected blood glucose value to the electronic device 200, and the electronic device 200 may output the blood glucose value in a manner of display, voice broadcast, vibration, or the like, so that the user learns a blood glucose status of the user.

In a specific implementation, after the device is activated, the electronic device 100 may send a broadcast signal to surroundings, to find the surrounding electronic device 200 that can be connected, and further establishes a communication connection to the electronic device 200.

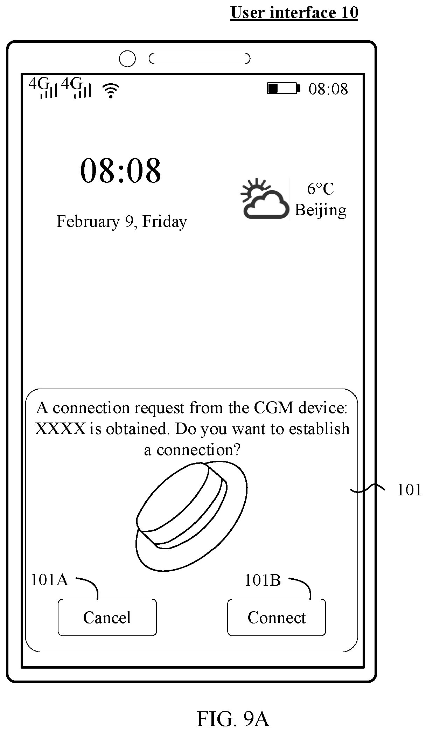

**For example,** **FIG. 9A to FIG. 9D** **are related user interfaces on the electronic device 200 in a process of establishing a connection between the electronic device 200 and the electronic device 100 by using an example in which the electronic device 100 is a CGM device.**

FIG. 9A shows a user interface 10 displayed after the electronic device 200 obtains the broadcast signal sent by the electronic device 100.

As shown in FIG. 9A, the user interface 10 may include a window 101, where the window 101 may be used to prompt the user that a CGM device requests to establish a connection.

The window 101 may include a cancel option 101A and a connection option 101B. The cancel option 101A may be used to reject to establish a connection to the electronic device 100, and the connection option 101B may be used to agree to establish a connection to the electronic device 100.

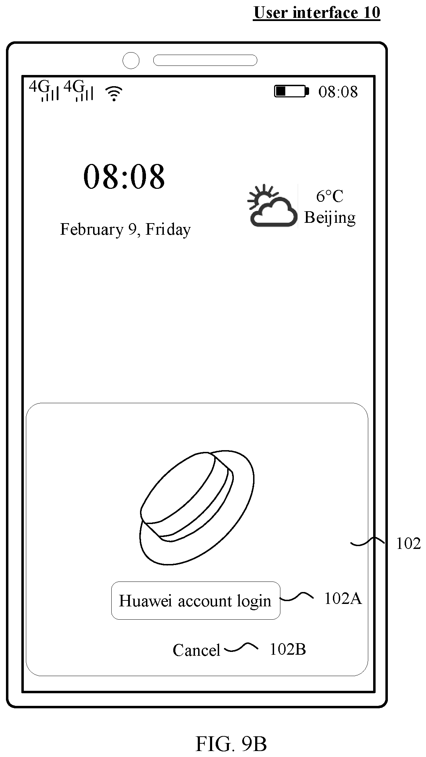

When the electronic device 200 detects a user operation performed on the connection option 101B, for example, a tap operation, the electronic device 200 may update the window 101 to a window 102 shown in FIG. 9B in response to the operation. The window 102 may prompt the user to log in to an account.

In an internal implementation, after the electronic device 200 detects the user operation performed on the connection option 101B, the electronic device 200 may send connection information to the electronic device 100. The connection information may be used to trigger the electronic device 100 to establish a connection to the electronic device 200.

The window 102 may include a Huawei account login option 102A and a cancel option 102B. The Huawei account login option 102A may be used to trigger account login by using a Huawei account, and the cancel option 102B may be used to cancel account login.

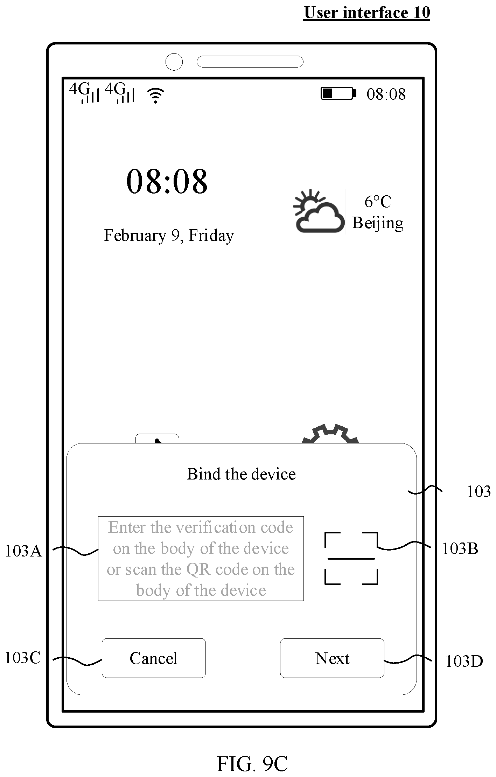

When the electronic device 200 detects a user operation performed on the Huawei account login option 102A, for example, a tap operation, in response to the operation, the electronic device 200 may establish a connection to the electronic device 100 by using the Huawei account, and update the window 102 to a window 103 shown in FIG. 9C. The window 103 may be used to prompt the user to bind the device.

It may be understood that FIG. 9B is an optional user interface. After the electronic device 100 detects the user operation performed on the connection option 101B shown in FIG. 9A, the electronic device 100 may update the window 101 to the window 103 shown in FIG. 9C in response to the operation.

As shown in FIG. 9C, the window 103 may include an input box 103A, a code scanning option 103B, a cancel option 103C, and a next option 103D. The input box 103A may be used to fill in a verification code on the electronic device 100, to trigger binding of the electronic device 100 to a Huawei account that is logged in to on the electronic device 200, so that the electronic device 100 can exchange data with the electronic device 200 on which the Huawei account is logged in to. The code scanning option 103B may be used to enable a code scanning function. After the electronic device 200 scans a QR code on the body of the electronic device 100, the electronic device 100 may also be triggered to be bound to the Huawei account that is logged in to on the electronic device 200.

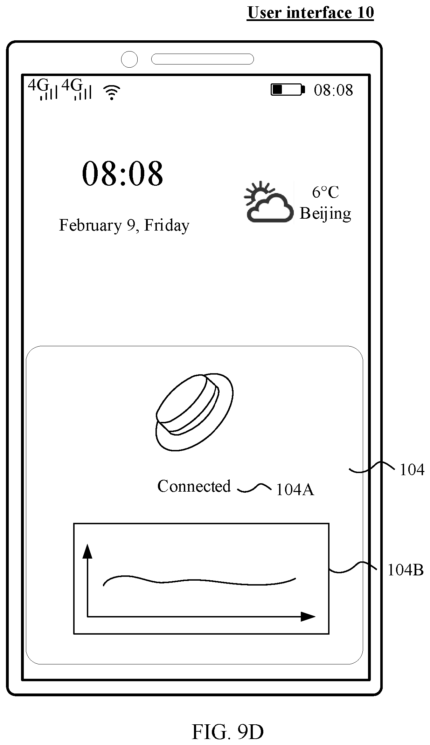

After the user enters the verification code or scans the QR code on the body of the electronic device 100, the electronic device 200 may detect a user operation performed on the next option 103D, for example, a tap operation. The electronic device 200 may complete binding of the electronic device 100, to establish the connection between the electronic device 100 and the electronic device 200, and update the window 103 to a window 104 shown in FIG. 9D. The window 104 may be used to prompt that the connection is successfully established.

As shown in FIG. 9D, the window 104 may include prompt information 104A, where the prompt information 104A may prompt the user that a connection has been established between the electronic device 100 and the electronic device 200.

Optionally, the window 104 may further include a blood glucose curve 104B. The blood glucose curve 104B may be used to display a curve obtained by connecting one or more blood glucose values sent by the electronic device 100 after the electronic device 100 establishes the connection to the electronic device 200. In this way, the user can learn a blood glucose fluctuation status of the user in a period of time.

It may be understood that the window 104 may also be configured to display a blood glucose value collected by the electronic device 100 in real time, a blood glucose change trend, and/or the like. In this way, the user can learn a current real-time blood glucose status of the user.

In addition, after the electronic device 100 establishes the communication connection to the electronic device 200, the electronic device 200 may display, on a desktop or a leftmost screen, the blood glucose value collected by the electronic device 100, or display, in a floating window, the blood glucose value collected by the electronic device 100. In this way, in a process of using the electronic device 200, the user may connect to a blood glucose status of the user at any time, and the displayed blood glucose value does not affect using the electronic device 200 by the user.

It can be learned from FIG. 9A to FIG. 9D that, after the electronic device 100 is activated, the electronic device 100 may directly broadcast a signal to the outside, to search for a device that can be connected. In this way, after wearing the electronic device 100, the user does not need to manually open a connection interface corresponding to the terminal device, and establish a connection to the electronic device 100 by performing complex operations. After obtaining the broadcast signal of the electronic device 100, the electronic device 200 may automatically pop up a pop-up window, and the user only needs to complete a communication connection between the electronic devices based on a prompt of the pop-up window. This facilitates an operation of the user, and quickly establishes the connection between the devices.

In addition, it should be noted that, after the device is worn, although the electronic device 100 may start to measure the body indicator of the user, real and accurate data of the user can be collected only after a period of time.

The CGM device is used as an example. After being placed under the skin, the sensor of the transmitter needs to interact electrochemically with glucose in the interstitial fluid, and a blood glucose value of the user is reflected by using a current formed through directional movement of electrons. Therefore, after the transmitter is worn, the electronic device 100 needs to wait for a period of time, for example, half an hour or one hour, to complete initialization of the device, so that the electronic device 100 can collect a stable and accurate blood glucose value.

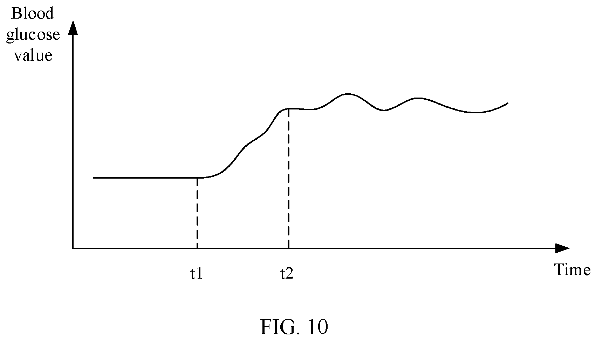

For example, FIG. 10 is a blood glucose change curve collected by the electronic device 100 after the electronic device 100 is worn on the user by using an example in which the electronic device 100 is a CGM.

As shown in FIG. 10, t1 is a time point at which the user wears the electronic device 100, t1-t2 is a period of time in which the electronic device 100 is initialized, and a phase after t2 is a phase in which the electronic device 100 normally collects a blood glucose value.

It can be learned that, after the user wears the electronic device 100, the electronic device 100 needs to be initialized for a period of time. A blood glucose value collected in the initialization phase cannot reflect a real blood glucose value of the user. The blood glucose value collected by the electronic device 100 can represent the real blood glucose value of the user only after initialization ends.

Therefore, to prevent the electronic device 100 from sending, to the electronic device 200, the blood glucose value collected in the initialization phase, the electronic device 200 generally starts countdown for preset duration, for example, half an hour or one hour, after establishing the connection to the electronic device 100, and after the electronic device 100 completes initialization, displays the blood glucose value collected by the electronic device 100.

However, when the electronic device 200 establishes the connection to the electronic device 100, actually, the electronic device 100 has been worn on the user, and initialization is performed for a period of time. If countdown starts after the connection is established, it is equivalent to that the user waits for one more period of time.

Based on an activation principle of the device activation circuit provided in this embodiment of this application, when the electronic device 100 is activated, the electronic device 100 is exactly in a phase in which the user wears the device or a preparation phase of wearing the device. Therefore, if the electronic device 100 completes device activation when the user wears the device, timing may start after the device is activated, or if the electronic device 100 completes device activation in the preparation phase of wearing the device, whether the user wears the electronic device 100 may be further determined with reference to a sensor such as a temperature sensor or an electrochemical sensor on the electronic device 100. After it is detected that the user wears the electronic device 100, timing starts. In this way, after the electronic device 100 establishes the connection to the electronic device 200, duration recorded by the electronic device 100 before the connection is established may be subtracted from the countdown duration of the electronic device 200, to shorten a waiting time of the user.

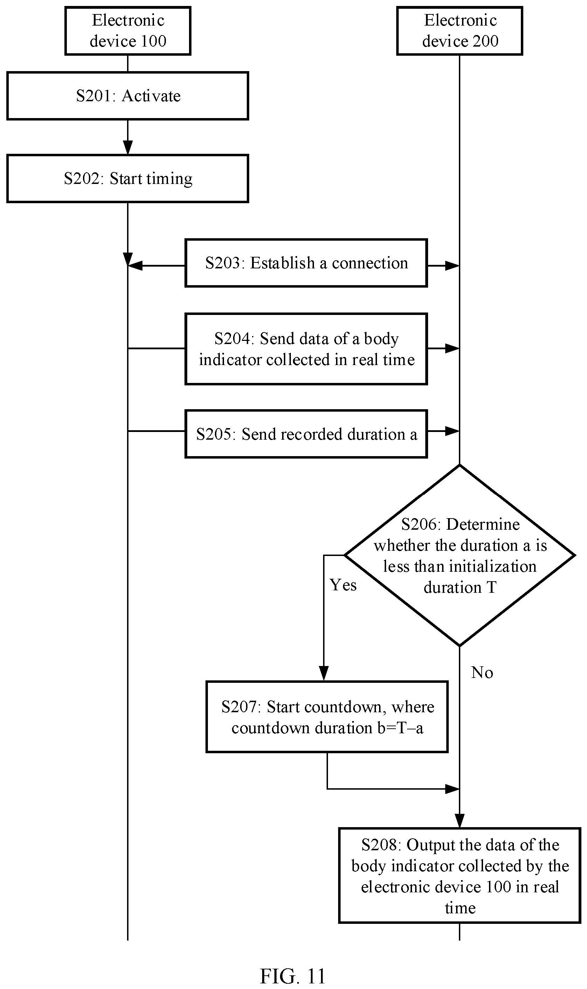

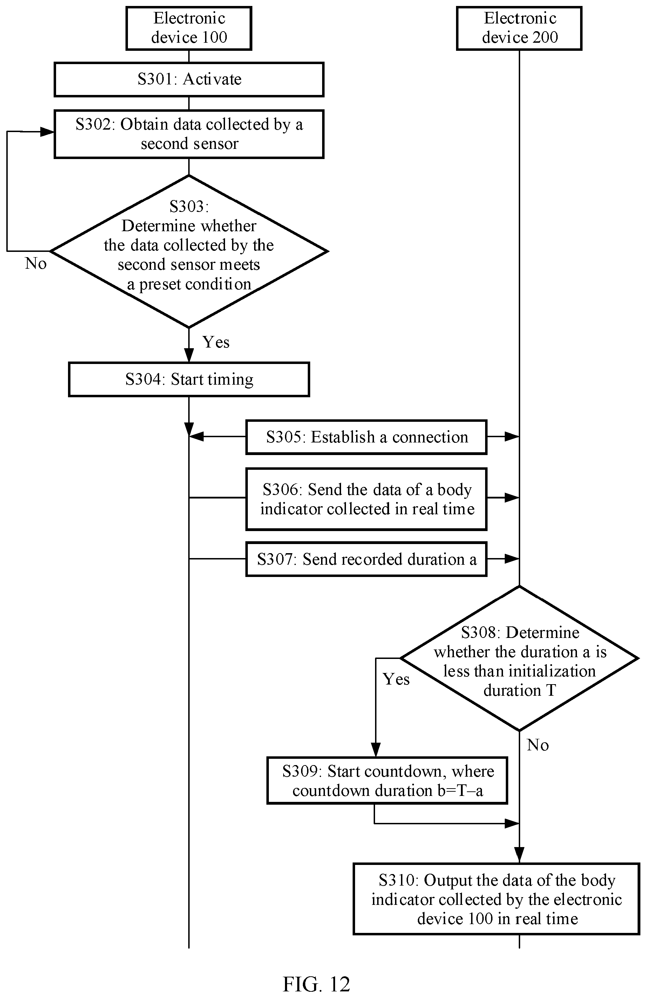

**With reference to** **FIG. 11** **and** **FIG. 12****, for two different cases of the device activation circuit, the following describes a process of interaction between the electronic device 100 and the electronic device 200 before the electronic device 200 outputs the data of the body indicator collected by the electronic device 100.**

FIG. 11 is a diagram of interaction between the electronic device 100 and the electronic device 200 before the electronic device 200 outputs the data of the body indicator collected by the electronic device 100 in real time when the electronic device 100 is activated when the user wears the electronic device 100.

S201: The electronic device 100 is activated.

The electronic device 100 may be activated by using the device activation circuit 110.

The device activation circuit 110 may include an optical sensor. If the electronic device 100 is the CGM device shown in FIG. 1, the optical sensor may be disposed on the side A of the transmitter in the electronic device 100 (with reference to FIG. 4); or the device activation circuit 110 may include a magnetic sensor. If the electronic device 100 is the CGM device shown in FIG. 1, a magnet may be placed in the implanter of the electronic device 100.

Under effect of the device activation circuit 110, the electronic device 100 may be activated when the user wears the electronic device 100.

For a specific principle of activating the device by the device activation circuit 110 when the user wears the device, refer to the related content in FIG. 5 to FIG. 7. Details are not described herein again.

**S202:** The electronic device 100 starts timing.

The electronic device 100 may start timing after being activated, and record duration experienced by the electronic device 100 from a time when the device is activated.

In a specific implementation, a timing module, for example, a real time clock (real time clock, RTC) chip, may be configured in the electronic device 100, and timing is performed by using the timing module.

**S203:** The electronic device 100 establishes a connection to the electronic device 200.

For example, after being activated, the electronic device 100 may send a broadcast signal, for example, a Bluetooth low energy (Bluetooth low energy, BLE) broadcast signal, to surroundings. After the electronic device 200 obtains the broadcast signal and sends a connection establishment agree message to the electronic device 100, the electronic device 100 and the electronic device 200 may complete establishment of the connection.

It may be understood that, after the electronic device 200 obtains the broadcast signal sent by the electronic device 100, the electronic device 200 may display a prompt to the user, to make the user select whether to agree to establish the connection to the electronic device 100. In addition, after detecting an operation that the user agrees to establish the connection, the electronic device 200 sends, to the electronic device 100, the connection establishment agree message.

For details of user interfaces displayed after the electronic device 200 receives the broadcast signal, refer to FIG. 9A to FIG. 9D. Details are not described herein again.

**S204:** The electronic device 100 sends the data of the body indicator collected in real time to the electronic device 200.

After being activated, the electronic device 100 may start to measure the body indicator of the user, and collect the data of the body indicator of the user.

For example, after establishing the connection to the electronic device 200, the electronic device 100 may start to send, to the electronic device 200, the data of the body indicator collected in real time; or the electronic device 100 may send, to the electronic device 200 after receiving, from the electronic device 200, a request for monitoring the body indicator, the data of the body indicator collected in real time; or the electronic device 100 may send, to the electronic device 200 after timing duration reaches initialization duration T, the data of the body indicator collected in real time. An execution occasion of step S204 is not limited in this embodiment of this application.

The initialization duration T is duration that needs to be spent from a time when the electronic device 100 starts to work to a time when the electronic device 100 collects stable data that can reflect a real body indicator of the user. The duration may be determined by a structure, a component, and a collected body indicator of the electronic device 100. Initialization duration required by different electronic devices may be different, and the initialization duration may be half an hour, one hour, or the like. In this embodiment of this application, the initialization duration may also be referred to as first preset duration.

It may be understood that step S204 may be performed before or after any one of steps S205 to S207. This is not limited in this embodiment of this application.

**S205:** The electronic device 100 sends recorded duration a to the electronic device 200.

After establishing the connection to the electronic device 200, the electronic device 100 may send, to the electronic device 200, the duration a that starts to be recorded by the electronic device 100 after the electronic device 100 is activated.

For example, after the electronic device 100 establishes the connection to the electronic device 200, the electronic device 100 may send the recorded duration a to the electronic device 200 in response to a message that is sent by the electronic device 200 and that is for requesting to obtain the timing duration. When needing to perform countdown, the electronic device 200 may send, to the electronic device 100, the message for requesting to obtain the timing duration.

It may be understood that an occurrence sequence of step S204 and step S205 does not limit a sequence of performing step S204 and step S205. For example, the electronic device 100 may first perform step S205 and then perform step S204, or simultaneously perform step S204 and step S205.

**S206:** The electronic device 200 determines whether the duration a is less than the initialization duration T.

If the duration a is greater than or equal to the initialization duration T, it indicates that duration from a time when the electronic device 100 is activated to a time when the electronic device 200 needs to perform countdown has been greater than or equal to the initialization duration. In this case, the data of the body indicator collected by the electronic device 100 has been stable and can be used to reflect a real body status of the user. Therefore, the electronic device 200 may perform step S208 to directly output the data of the body indicator collected by the electronic device 100, where the data may be data of the body indicator currently collected by the electronic device 100 in real time, or may be data of the body indicator collected by the electronic device 100 after the timing duration reaches T.

If the duration a is less than the initialization duration T, it indicates that duration from a time when the electronic device 100 is activated to a time when the electronic device 200 needs to perform countdown is still less than the initialization duration. In this case, the electronic device 100 is still in the initialization phase, and the collected data of the body indicator is unstable and cannot be used to reflect the real body status of the user. Therefore, the electronic device 200 may perform step S207 to perform countdown, and wait for the electronic device 100 to collect stable data of the body indicator.

**S207:** The electronic device 200 starts countdown, where countdown duration b=T-a.

Because the user has worn the electronic device 100 for a period of time before the electronic device 200 starts countdown, the electronic device 200 does not need to wait for complete initialization duration before outputting the data of the body indicator collected by the electronic device 100 in real time. In this way, the waiting time of the user can be reduced, and a speed at which the user views the body status of the user can be accelerated.

For example, after calculating the countdown duration, the electronic device 200 may display countdown, so that the user knows a length of duration that the user needs to wait for.

**S208:** The electronic device 200 outputs the data of the body indicator collected by the electronic device 100 in real time.

After the electronic device 100 undergoes the initialization phase, the electronic device 100 can collect stable data of the body indicator of the user, and the data can reflect the real body status of the user. Therefore, the electronic device 100 may send the data of the body indicator collected by the electronic device 100 to the electronic device 200, and the electronic device 200 outputs the data, so that the user learns a current real body status of the user.

It may be understood that the electronic device 200 may send, to the electronic device 100 after determining that the duration a is greater than the initialization duration T, or after countdown ends, a message for requesting to monitor the body indicator of the user. The electronic device 100 may send, to the electronic device 200 in response to the message, the data of the body indicator collected in real time.

The electronic device 200 may output the data of the body indicator of the user in a manner of display, voice broadcast, vibration, or the like. A manner in which the electronic device 200 outputs the data of the body indicator is not limited in this embodiment of this application. In addition, the electronic device 200 may display only a value that is of the body indicator and that is updated in real time. In this way, the user can view a current body status in real time. Alternatively, the electronic device 200 may draw a continuous body indicator curve with reference to historically collected data and data that is collected in real time and display the curve. In this way, the user can view a fluctuation status of the body indicator of the user in a period of time. A form in which the electronic device 200 outputs the data of the body indicator is not limited in this embodiment of this application.

It can be learned from steps S201 to S208 that, under effect of the device activation circuit 110, the electronic device 100 is activated when the user wears the electronic device 100, to facilitate the electronic device 100 in monitoring a time point at which the user wears the device. Therefore, the time that the electronic device 100 experiences before establishing the connection to the electronic device 200 is considered, to omit or shorten duration of countdown of the electronic device 200, thereby reducing waiting duration of the user.

In addition, FIG. 12 is a diagram of interaction between the electronic device 100 and the electronic device 200 before the electronic device 200 outputs the data of the body indicator collected by the electronic device 100 in real time when the electronic device 100 is activated before the user wears the electronic device 100.

**S301:** The electronic device 100 is activated.

The electronic device 100 may be activated by using the device activation circuit 110.

The device activation circuit 110 may include an optical sensor. If the electronic device 100 is the CGM device shown in FIG. 1, the optical sensor may be disposed on the side B of the transmitter in the electronic device 100 (with reference to FIG. 4); or the device activation circuit 110 may include a magnetic sensor. If the electronic device 100 is the CGM device shown in FIG. 1, a magnet may be placed in the packaging cover of the electronic device 100.

Under effect of the device activation circuit 110, the electronic device 100 may be activated before the user wears the electronic device 100.

For a specific principle of activating the device by the device activation circuit 110 before the user wears the device, refer to the related content in FIG. 5 to FIG. 7. Details are not described herein again.

**S302:** The electronic device 100 obtains data collected by a second sensor.

There may be one or more second sensors, and the electronic device 100 may determine the body indicator of the user based on the data collected by the second sensor. In other words, the electronic device 100 may determine, based on the data collected by the second sensor, whether the user wears the electronic device 100.

The electronic device 100 is activated before the electronic device 100 is worn. Therefore, after the electronic device 100 is activated, the electronic device 100 may start to monitor an event that the user wears the electronic device 100, so that the electronic device 100 starts timing after the user wears the electronic device 100.

After the user wears the electronic device 100, the data collected by the second sensor changes accordingly. Therefore, the electronic device 100 may determine, by using the data collected by the second sensor, whether the user wears the electronic device 100.

For example, the second sensor may include an electrochemical sensor, a temperature sensor, and the like. This is not limited in this embodiment of this application.

For example, the electronic device 100 is a CGM device. After the user wears the electronic device 100, the electrochemical sensor is implanted into the user. The electrochemical sensor may be configured to detect a current value under the skin, so that the electronic device 100 calculates a blood glucose value based on the current value. The temperature sensor may be configured to detect a temperature of a surrounding environment. The electronic device 100 may correct, based on the temperature, the blood glucose value calculated by the electronic device 100, so that the blood glucose value calculated by the electronic device 100 is more accurate.

**S303:** The electronic device 100 determines whether the data collected by the second sensor meets a preset condition.

If the second sensor includes an electrochemical sensor, the preset condition may include that a current value collected by the electrochemical sensor is within a preset range. If the second sensor further includes a temperature sensor, the preset condition further includes that a temperature value collected by the temperature sensor is within a preset range.

If the data collected by the second sensor meets the preset condition, it indicates that the user wears the electronic device 100. Therefore, the electronic device 100 may perform step S304 to start timing. Otherwise, it indicates that the user does not wear the electronic device 100. The electronic device 100 may perform step S302 to continue to obtain the data collected by the second sensor, and monitor the event that the user wears the electronic device 100.

It should be noted that, in a process in which the user prepares to wear the electronic device 100, the user may forget to wear the electronic device 100, or wear the electronic device 100 at an interval of a long time. For the electronic device 100 that needs to be successfully worn only after the second sensor is implanted under the skin of the user, for example, a CGM device, the electronic device 100 usually needs to be kept in a sterile state. If the user forgets to wear the electronic device 100, or spends a long time from preparing to wear the electronic device 100 to wearing the electronic device 100, a risk of infection of the electronic device 100 is increased. If the electronic device 100 is contaminated, after the user wears the electronic device 100, a risk that the user is infected is increased, and body health of the user is affected.

Therefore, if the electronic device 100 is activated before being worn by the user, the electronic device 100 may determine, with reference to the data collected by the second sensor in step S302, whether prompt information needs to be output, to remind the user to wear the electronic device 100 in a timely manner.

For example, using an example in which the second sensor includes a temperature sensor and an electrochemical sensor, the following three scenarios may exist:
(1) A current value collected by the electrochemical sensor is 0, and a temperature value collected by the temperature sensor is room temperature (for example, less than 30°C) and does not change significantly.

In this scenario, it indicates that the user does not wear the electronic device 100 at the moment. Therefore, the electronic device 100 may output the prompt information (first prompt information) at a regular interval, for example, at an interval of 10 minutes, to prompt the user to wear the electronic device 100.

Further, if the user does not wear the electronic device 100 after the electronic device 100 is activated for preset duration (for example, 12 hours), the electronic device 100 may remind the user that the device is unavailable and cannot be worn, and automatically turn off a power supply, to reduce a risk of infection of the user.

(2) A current value collected by the electrochemical sensor is 0, and a temperature value collected by the temperature sensor changes rapidly from room temperature, and is close to the skin temperature (for example, 30°C) of the human epidermis.

In this scenario, it indicates that the user has worn the electronic device 100, but the second sensor may fail to be implanted, for example, the second sensor is not implanted under the skin. Therefore, the electronic device 100 may output prompt information (for example, second prompt information), to prompt the user that implantation may fail, and to check an implantation status of the device.

Further, if the user repeatedly wears the electronic device 100 for a plurality of times, and after the electronic device 100 reminds the user of an implantation failure for a plurality of times, the electronic device 100 may remind the user to replace the second sensor.

(3) A current value collected by the electrochemical sensor changes, and a temperature value collected by the temperature sensor also changes.

In this scenario, it indicates that the electronic device 100 is successfully worn, and the electronic device 100 may perform step S304 to start timing.

It may be understood that if the electronic device 100 further includes another sensor, the electronic device 100 may further determine, with reference to data collected by the another sensor, a status of wearing the electronic device 100 by the user currently. This is not limited in this embodiment of this application. In addition, if the electronic device 100 can be activated when being worn by the user, the electronic device 100 may also determine the foregoing scenario (1) and scenario (2) based on the data collected by the second sensor, and output corresponding prompt information when the data collected by the second sensor meets the preset condition.

It should be noted that, in the scenario (1) and the scenario (2), if a communication connection is established between the electronic device 100 and the electronic device 200, in addition to outputting the prompt information by the electronic device 100, the electronic device 100 may send the prompt information to the electronic device 200, and the electronic device 200 outputs the prompt information. In this way, the user can view, by using the electronic device 200, prompt information that prompts the user to wear the electronic device 100 in a timely manner or that the electronic device 100 fails to be worn.

**S304:** The electronic device 100 starts timing.

**S305:** The electronic device 100 establishes a connection to the electronic device 200.

**S306:** The electronic device 100 sends the data of the body indicator collected in real time to the electronic device 200.

**S307:** The electronic device 100 sends recorded duration a to the electronic device 200.

**S308:** The electronic device 200 determines whether the duration a is less than an initialization duration T.

If the duration a is less than the initialization duration T, the electronic device 200 performs step S309; and otherwise, the electronic device 100 performs step S310.

**S309:** The electronic device 200 starts countdown, where countdown duration b=T-a.

**S310:** The electronic device 200 outputs the data of the body indicator collected by the electronic device 100 in real time.

It may be understood that content described in steps S304 to S310 is similar to that described in steps S202 to S208. For detailed content of steps S304 to S310, refer to the foregoing steps S202 to S208. Details are not described herein again.

It can be learned from steps S301 to S310 that, under effect of the device activation circuit 110, the electronic device 100 is activated before the user wears the electronic device 100. Therefore, after being activated, the electronic device 100 may start to monitor an event that the user wears the electronic device 100, so as to record, in a timely manner, a time point at which the user wears the electronic device 100. The time that the electronic device 100 experiences before establishing the connection to the electronic device 200 is considered, to omit or shorten duration of countdown of the electronic device 200, thereby reducing waiting duration of the user.

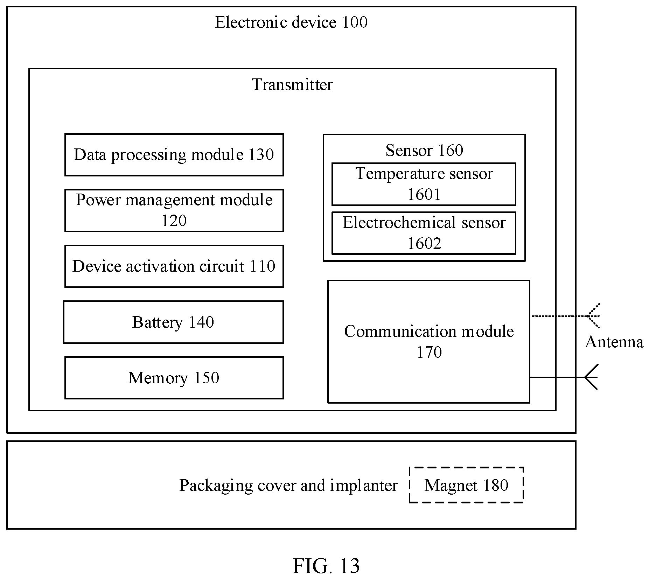

**FIG. 13** **is a diagram of an overall structure of an electronic device 100 according to an embodiment of this application.**

As shown in FIG. 13, the electronic device 100 may include a transmitter, along with two parts: a packaging cover and an implanter. The transmitter may include hardware such as a data processing module 130, a power management module 120, a device activation circuit 110, a battery 140, a memory 150, a sensor 160, and a communication module 170.

The device activation circuit 110 may include a sensing module 111 and a switching module 112.

The data processing module 130 may include one or more processing units. For example, the data processing module 130 may include a modem processor, a digital signal processor, a controller, a baseband processor, a neural network processor, and the like. Different processing units may be independent components, or may be integrated into one or more processors. The data processing module 130 may also be referred to as a processor.

For specific related descriptions of the device activation circuit 110, the data processing module 130, the power management module 120, and the battery 140, refer to the related content in FIG. 1. Details are not described herein again.

If the sensing module 111 in the device activation circuit 110 includes a magnetic sensor, the packaging cover or the implanter of the electronic device 100 may include a magnet 180.

The memory 150 may be configured to store data collected by the sensor 160, and data obtained through calculation after the data processing module 130 processes the data collected by the sensor 160, for example, data of a body indicator of a user.

The sensor 160 may include one or more sensors, for example, a temperature sensor 1601 and an electrochemical sensor 1602.

The temperature sensor 1601 may be configured to detect a temperature. In some implementations, the electronic device 100 may determine a skin temperature of the user and/or an ambient temperature based on the temperature detected by the temperature sensor 1601.

The electrochemical sensor 1602 may be configured to detect a concentration of glucose. In some implementations, the electrochemical sensor 1602 may determine the concentration of the glucose by detecting oxygen consumption under a catalytic action of glucose oxidase or H₂O₂ that is generated by a glucose oxidation reaction in interstitial fluid. In some embodiments, the electrochemical sensor 1602 connects the glucose oxidase to an electrode surface by using an electronic medium such as a nanomaterial, metal osmium, ferrocene, or quinones, then implements electron transfer through a series of oxidation-reduction reactions, and further determines the concentration of the glucose.

The communication module 170 may provide a wireless communication solution that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The communication module 170 may be one or more components integrating at least one communication processing module. The communication module 170 receives an electromagnetic wave through an antenna, performs frequency modulation and filtering processing on an electromagnetic wave signal, and sends a processed signal to the data processing module 130. The communication module 170 may further receive a to-be-sent signal from the data processing module 130, perform frequency modulation and amplification on the to-be-sent signal, and convert the signal into an electromagnetic wave for radiation through the antenna.

In some embodiments, the electronic device 100 may send, by using the communication module 170, a broadcast signal to surroundings, receive information that is sent by another device, for example, the electronic device 200, and that is used to agree to establish a communication connection, and send, to the electronic device 200, data that is of a body indicator and that is collected in real time, and the like.

It may be understood that the structure shown in this embodiment of the present invention does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

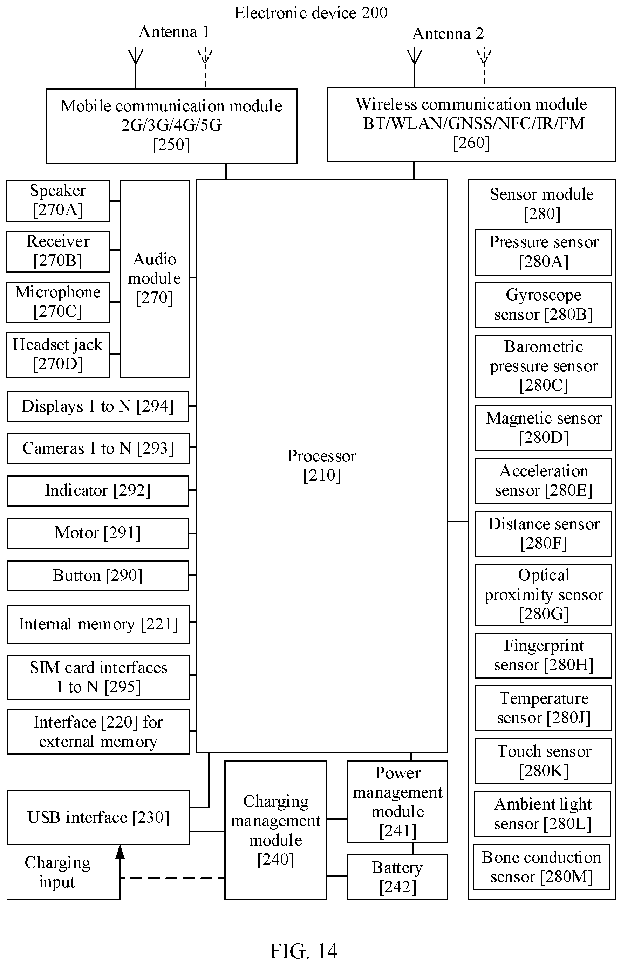

**FIG. 14** **is a diagram of a hardware structure of an electronic device 200 according to an embodiment of this application.**

The electronic device 200 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, an in-vehicle device, a smart home device, and/or a smart city device, and a specific type of the electronic device is not limited in this embodiment of this application.

The electronic device 200 may include a processor 210, an interface 220 for external memory, an internal memory 221, a universal serial bus (universal serial bus, USB) interface 230, a charging management module 240, a power management module 241, a battery 242, an antenna 1, an antenna 2, a mobile communication module 250, a wireless communication module 260, an audio module 270, a speaker 270A, a receiver 270B, a microphone 270C, a headset jack 270D, a sensor module 280, a button 290, a motor 291, an indicator 292, a camera 293, a display 294, a subscriber identity module (subscriber identification module, SIM) card interface 295, and the like. The sensor module 280 may include a pressure sensor 280A, a gyroscope sensor 280B, a barometric pressure sensor 280C, a magnetic sensor 280D, an acceleration sensor 280E, a distance sensor 280F, an optical proximity sensor 280G, a fingerprint sensor 280H, a temperature sensor 280J, a touch sensor 280K, an ambient light sensor 280L, a bone conduction sensor 280M, and the like.

It may be understood that the structure shown in this embodiment of the present invention does not constitute a specific limitation on the electronic device 200. In some other embodiments of this application, the electronic device 200 may include more or fewer components than those shown in the figure, or some components may be combined, or some components may be split, or different component arrangements may be used. The components shown in the figure may be implemented by hardware, software, or a combination of software and hardware.

**The processor 210** may include one or more processing units. For example, the processor 210 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

**In some implementations, the processor 210 may be configured to: determine whether timing duration a of the electronic device 100 is greater than initialization duration T, and perform countdown when the timing duration a is less than the initialization duration T, where countdown duration b=T-a. For specific descriptions of the timing duration a and the initialization duration T, refer to related content in** **FIG. 11** **and** **FIG. 12****. Details are not described herein again.**

The controller may generate an operation control signal based on an instruction operation code and a timing signal, to complete control of instruction fetching and instruction execution.

A memory may be further disposed in the processor 210, and is configured to store instructions and data. In some embodiments, the memory in the processor 210 is a cache memory. The memory may store an instruction or data that has been used or cyclically used by the processor 210. If the processor 210 needs to use the instruction or the data again, the processor may directly invoke the instruction or the data from the memory. This avoids repeated access, reduces waiting time of the processor 210, and therefore improves system efficiency.

A wireless communication function of the electronic device 200 may be implemented through the antenna 1, the antenna 2, the mobile communication module 250, the wireless communication module 260, the modem processor, the baseband processor, and the like.

The antenna 1 and the antenna 2 are configured to transmit and receive an electromagnetic wave signal. Each antenna in the electronic device 200 may be configured to cover one or more communication bands. Different antennas may be further reused, to improve antenna utilization. For example, the antenna 1 may be reused as a diversity antenna of a wireless local area network. In some other embodiments, the antenna may be used in combination with a tuning switch.

**The mobile communication module 250** may provide a wireless communication solution that is applied to the electronic device 200 and that includes 2G/3G/4G/5G or the like. The mobile communication module 250 may include at least one filter, a switch, a power amplifier, a low noise amplifier (low noise amplifier, LNA), and the like. The mobile communication module 250 may receive an electromagnetic wave through the antenna 1, perform processing such as filtering or amplification on the received electromagnetic wave, and transmit the electromagnetic wave to the modem processor for demodulation. The mobile communication module 250 may further amplify a signal modulated by the modem processor, and convert the signal into an electromagnetic wave for radiation through the antenna 1. In some embodiments, at least some functional modules in the mobile communication module 250 may be disposed in the processor 210. In some embodiments, at least some functional modules of the mobile communication module 250 may be disposed in a same device as at least some modules of the processor 210.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 270A, the receiver 270B, or the like), or displays an image or a video through the display 294. In some embodiments, the modem processor may be an independent component. In some other embodiments, the modem processor may be independent of the processor 210, and is disposed in a same device as the mobile communication module 250 or another functional module.

**The wireless communication module 260** may provide a wireless communication solution that is applied to the electronic device 200 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 260 may be one or more components integrating at least one communication processing module. The wireless communication module 260 receives an electromagnetic wave through the antenna 2, performs demodulation and filtering processing on an electromagnetic wave signal, and sends a processed signal to the processor 210. The wireless communication module 260 may further receive a to-be-sent signal from the processor 210, perform frequency modulation and amplification on the signal, and convert the signal into an electromagnetic wave for radiation through the antenna 2.

In some embodiments, in the electronic device 200, the antenna 1 is coupled to the mobile communication module 250, and the antenna 2 is coupled to the wireless communication module 260, so that the electronic device 200 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or satellite based augmentation systems (satellite based augmentation systems, SBAS).

**In some implementations, the electronic device 200 may establish a communication connection to the electronic device 100 by using the mobile communication module 150 or the wireless communication module 160, to obtain data that is of a body indicator and that is collected by the electronic device 100 in real time, the duration a recorded by the electronic device 100 starting from activation, and the like.**

**In this embodiment of this application, the mobile communication module 150 and/or the wireless communication module 160 may also be referred to as a communication module.**

The electronic device 200 implements a display function through the GPU, the display 294, the application processor, and the like. The GPU is a microprocessor for image processing, and is connected to the display 294 and the application processor. The GPU is configured to: perform mathematical and geometric computation, and render an image. The processor 210 may include one or more GPUs that execute program instructions to generate or change display information.

**The display 294** is configured to display an image, a video, and the like. In some embodiments, the electronic device 200 may include one or N displays 294, where N is a positive integer greater than 1.

**In some implementations, the electronic device 200 may display, by using the display 294, a related user interface used for establishing a connection to the electronic device 100, and data that is of a body indicator and that is collected by the electronic device 100. For details of related user interfaces that are displayed by the electronic device 200 and that are used for establishing the connection to the electronic device 100, refer to** **FIG. 9A to FIG. 9D****.**

The electronic device 200 may implement a shooting function through the ISP, the camera 293, the video codec, the GPU, the display 294, the application processor, and the like.

The camera 293 is configured to capture a static image or a video. In some embodiments, the electronic device 200 may include one or N cameras 293, where N is a positive integer greater than 1.

**In some implementations, in a process of establishing a connection between the electronic device 200 and the electronic device 100, the electronic device 200 may scan a QR code on a body of the electronic device 100 by using the camera 293, to bind the electronic device 100 to a system account that is logged in to on the electronic device 200.**

The internal memory 221 may include one or more random access memories (random access memory, RAM), and one or more non-volatile memories (non-volatile memory, NVM).

The random access memory may be directly read and written by the processor 210, may be configured to store executable programs (for example, machine instructions) of an operating system or another running program, and may also be configured to store data of a user and an application, and the like.

The non-volatile memory may also store executable programs, data of the user and the application, and the like, and may be loaded into the random access memory in advance, to be directly read and written by the processor 210.

**In some implementations, the internal memory 221 may be configured to store data that is of a body indicator and that is collected by the electronic device 100 and duration recorded by the electronic device 100.**

The electronic device 200 may implement an audio function, for example, music playing and recording, through the audio module 270, the speaker 270A, the receiver 270B, the microphone 270C, the headset jack 270D, the application processor, and the like.

The audio module 270 is configured to convert digital audio information into an analog audio signal for output, and is also configured to convert an analog audio input into a digital audio signal. The audio module 270 may be further configured to encode and decode an audio signal. In some embodiments, the audio module 270 may be disposed in the processor 210, or some functional modules in the audio module 270 are disposed in the processor 210.

The speaker 270A, also referred to as a "loudspeaker", is configured to convert an audio electrical signal into a sound signal. The electronic device 200 may listen to music or answer a hands-free call by using the speaker 270A.

**In some implementations, the electronic device 200 may broadcast, by using the speaker 270A, data that is of a body indicator and that is collected by the electronic device 100.**

The touch sensor 280K is also referred to as a "touch component". The touch sensor 280K may be disposed on the display 294, and the touch sensor 280K and the display 294 constitute a touchscreen, which is also referred to as a "touch screen". The touch sensor 280K is configured to detect a touch operation performed on or near the touch sensor. The touch sensor may transfer the detected touch operation to the application processor to determine a type of the touch event. A visual output related to the touch operation may be provided through the display 294. In some other embodiments, the touch sensor 280K may also be disposed on a surface of the electronic device 200 at a position different from that of the display 294.

**In some implementations, the electronic device 200 may detect an operation of a user by using the touch sensor 280K, and establish a communication connection to the electronic device 100 based on the operation of the user.**

The motor 291 may generate a vibration prompt. The motor 291 may be configured to provide an incoming call vibration prompt, or may be configured to provide a touch vibration feedback. For example, touch operations performed on different applications (for example, photographing and audio playing) may correspond to different vibration feedback effects. The motor 291 may also correspond to different vibration feedback effects for touch operations performed on different areas of the display 294. Different application scenarios (for example, a time reminder, information receiving, an alarm clock, and a game) may also correspond to different vibration feedback effects. Touch vibration feedback effect may be further customized.

**In some implementations, when outputting data that is of a body indicator and that is collected by the electronic device 100, the electronic device 200 may generate vibration by using the motor 291, to prompt the user to view the data of the body indicator collected by the electronic device 100.**

**In this embodiment of this application, the display 194, the motor 291, and the speaker 270A may also be referred to as output modules.**

The electronic device may be a portable terminal device carrying Harmony, iOS, Android, Microsoft, or another operating system, for example, a mobile phone, a tablet computer, or a wearable device; or may be a non-portable terminal device, for example, a laptop (Laptop) computer having a touch-sensitive surface or a touch panel, or a desktop computer having a touch-sensitive surface or a touch panel. A software system of the electronic device 200 may use a layered architecture, an event-driven architecture, a microkernel architecture, a microservice architecture, or a cloud architecture. In an embodiment of the present invention, a software structure of the electronic device 200 is described by using an Android system with a layered architecture as an example.

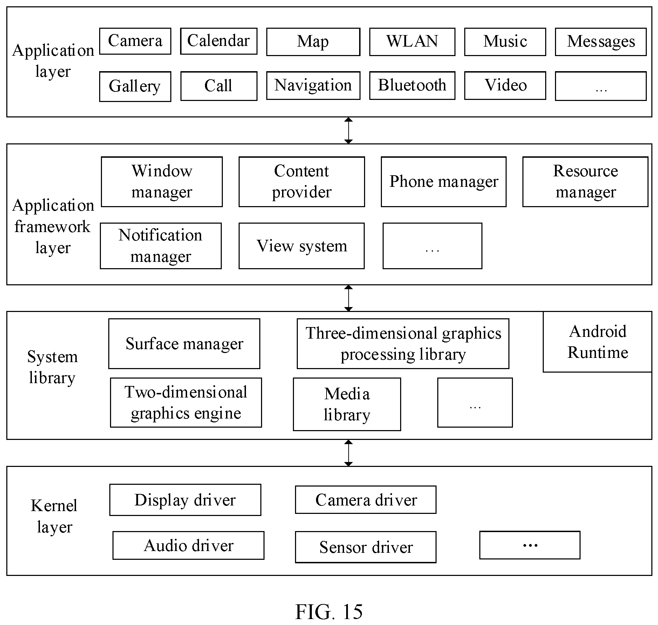

**FIG. 15** **is a block diagram of a software structure of an electronic device 200 according to an embodiment of this application.**

In the layered architecture, software is divided into several layers, and each layer has a clear role and task. The layers communicate with each other through a software interface. In some embodiments, the Android system is divided into four layers: an application layer, an application framework layer, an Android Runtime (Android Runtime) and a system library, and a kernel layer from top to bottom.

The application layer may include a series of application packages.

As shown in FIG. 15, the application packages may include applications such as Camera, Gallery, Calendar, Call, Map, Navigation, WLAN, Bluetooth, Music, Video, and Messages.

The application framework layer provides an application programming interface (application programming interface, API) and a programming framework for an application at the application layer. The application framework layer includes some predefined functions.

As shown in FIG. 15, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessible to an application. The data may include a video, an image, audio, calls made and answered, a browse history and a bookmark, a personal address book, and the like.

The view system includes visual controls such as a text display control and a picture display control. The view system may be configured to construct an application. A display interface may include one or more views. For example, a display interface including a message notification icon may include a text display view and a picture display view.

The phone manager is configured to provide a communication function for the electronic device 200, for example, management of a call status (including answering, declining, or the like).

The resource manager provides various resources such as a localized character string, an icon, a picture, a layout file, and a video file for an application.

The notification manager enables an application to display, in the status bar, notification information, which may be used for conveying a notification-type message that may automatically disappear after a short stay without user interaction. For example, the notification manager is configured to: notify download completion, give a message notification, and the like. The notification manager may alternatively be a notification that appears in a top status bar of the system in a form of a chart or scroll bar text, for example, a notification of an application running on the background or a notification that appears on the screen in a form of a dialog window. For example, text information is prompted in the status bar, an alert tone is made, the electronic device vibrates, or an indicator blinks.

The Android Runtime includes a core library and a virtual machine. The Android Runtime is responsible for scheduling and management of the Android system.

The core library includes two parts: a function that needs to be called in Java language and a core library of Android.

The application layer and the application framework layer run in the virtual machine. The virtual machine executes Java files of the application layer and the application framework layer as binary files. The virtual machine is configured to execute functions such as object life cycle management, stack management, thread management, security and exception management, and garbage collection.

The system library may include a plurality of functional modules, for example, a surface manager (surface manager), media libraries (Media Libraries), a three-dimensional graphics processing library (for example, an OpenGL ES), a 2D graphics engine (for example, an SGL), and the like.

The surface manager is configured to: manage a display subsystem and provide fusion of 2D and 3D layers for a plurality of applications.

The media library supports playback and recording in a plurality of commonly used audio and video formats, and static image files. The media library may support a plurality of audio and video coding formats, for example, MPEG 4, H.264, MP3, AAC, AMR, JPG, and PNG.

The three-dimensional graphics processing library is configured to implement three-dimensional graphics drawing, image rendering and compositing, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes working procedures of software and hardware of the electronic device 200 by using examples with reference to a photo capture scenario.

When the touch sensor 280K receives a touch operation, a corresponding hardware interruption is sent to the kernel layer. The kernel layer processes the touch operation into an original input event (including information such as coordinates of touch and a timestamp of the touch operation). The original input event is stored at the kernel layer. The application framework layer obtains the original input event from the kernel layer, and identifies a control corresponding to the input event. Using an example in which the touch operation is a touch tapping operation, and a control corresponding to the tapping operation is a control of an icon of the camera application, the camera application invokes an interface of the application framework layer, and the camera application is started, so that a camera driver is started by invoking the kernel layer, and a static image or a video is captured by using the camera 293.

It should be understood that the steps in the foregoing method embodiments may be completed by using a hardware integrated logic circuit or instructions in a form of software in the processor. The steps of the method disclosed with reference to embodiments of this application may be directly performed by a hardware processor, or may be performed by a combination of hardware and software modules in a processor.

This application further provides an electronic device, and the electronic device may include a memory and a processor. The memory may be configured to store a computer program. The processor may be configured to invoke the computer program in the memory, so that the electronic device performs the method performed by the electronic device 100 or the electronic device 200 in any one of the foregoing embodiments.

This application further provides a chip system. The chip system includes at least one processor, configured to implement functions in the method performed by the electronic device 100 or the electronic device 200 in any one of the foregoing embodiments.

In a possible design, the chip system further includes a memory. The memory is configured to store program instructions and data. The memory is located inside the processor or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When implemented by using software, the processor may be a general-purpose processor, and is implemented by reading software code stored in the memory.

Optionally, there may also be one or more memories in the chip system. The memory may be integrated with the processor, or may be separated from the processor. This is not limited in this embodiment of this application. For example, the memory may be a non-transitory processor such as a read only memory ROM, and the memory and the processor may be integrated on a same chip, or may be disposed on separate chips respectively. A type of the memory and an arrangement manner of the memory and the processor are not limited in this embodiment of this application.

For example, the chip system may be a field programmable gate array (field programmable gate array, FPGA), an application-specific integrated circuit (application-specific integrated circuit, ASIC), a system on chip (system on chip, SoC), a central processing unit (central processor unit, CPU), a network processor (network processor, NP), a digital signal processor (digital signal processor, DSP), a microcontroller unit (microcontroller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform the method performed by the electronic device 100 or the electronic device 200 in any one of the foregoing embodiments.

This application further provides a computer-readable storage medium. The computer-readable storage medium stores a computer program (which may also be referred to as code or instructions). When the computer program is run, a computer is enabled to perform the method performed by the electronic device 100 or the electronic device 200 in any one of the foregoing embodiments.

It should be understood that, the processor in this embodiment of this application may be an integrated circuit chip, and has a signal processing capability. In an implementation process, the steps in the foregoing method embodiments may be completed by using an integrated logic circuit of hardware in the processor or instructions in a form of software. The processor may be a general-purpose processor, a digital signal processor (digital signal processor, DSP), an application-specific integrated circuit (application-specific integrated circuit, ASIC), a field programmable gate array (field programmable gate array, FPGA) or another programmable logic device, a discrete gate or a transistor logic device, or a discrete hardware component. The methods, steps, and logic block diagrams disclosed in embodiments of this application may be implemented or executed. The general-purpose processor may be a microprocessor, or the processor may be any conventional processor or the like. The steps of the methods disclosed with reference to embodiments of this application may be directly performed by a hardware decoding processor, or may be performed by using a combination of hardware and software modules in the decoding processor. The software module may be located in a mature storage medium in the art, such as a random access memory, a flash memory, a read-only memory, a programmable read-only memory, an electrically erasable programmable memory, or a register. The storage medium is located in the memory, and the processor reads information in the memory and completes the steps in the foregoing method in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by one or more processors, the apparatus is enabled to perform the methods in the foregoing method embodiments.

The apparatus, the computer-readable storage medium, the computer program product, or the chip provided in embodiments of this application is configured to perform the corresponding methods provided above. Therefore, for beneficial effects that can be achieved, refer to beneficial effects in the corresponding methods provided above, and details are not described herein again.

The implementations of this application may be randomly combined to achieve different technical effects.

All or some of the foregoing embodiments may be implemented by using software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, the procedures or functions according to this application are all or partially generated. The computer may be a general-purpose computer, a dedicated computer, a computer network, or other programmable apparatuses. The computer instructions may be stored in a computer-readable storage medium, or transmitted from one computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, wireless, or microwaves) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or some of the procedures of the methods in the foregoing embodiments may be implemented by a computer program instructing related hardware. The program may be stored in the computer-readable storage medium. When the program is executed, the procedures in the foregoing method embodiments may be included. The foregoing storage medium includes any medium that can store program code, such as a ROM, a random access memory RAM, a magnetic disk, or an optical disc.

In conclusion, the foregoing is merely embodiments of the technical solution of the present invention, but is not intended to limit the protection scope of the present invention. Any modification, equivalent replacement, or improvement made according to the disclosure of the present invention shall fall within the protection scope of the present invention.

## Claims

1. A first electronic device, wherein the first electronic device is configured to measure a body indicator of a user, the first electronic device comprises an activation circuit, a power management module, a battery, and a data processing module, the activation circuit comprises a first end and a second end, the first end is connected to the power management module, the second end is connected to the battery, and the power management module is connected to the data processing module;
the activation circuit is configured to determine whether the user wears the first electronic device or whether the user prepares to wear the first electronic device;
when the activation circuit determines that the user wears the first electronic device or the user prepares to wear the first electronic device, a connection between the first end and the second end is switched from being cut off to being conducted;
the battery is configured to supply power to the data processing module by using the power management module after the connection between the first end and the second end is conducted; and
the data processing module is configured to determine the body indicator of the user based on data obtained by the first electronic device.

2. The first electronic device according to claim 1, wherein the data processing module, when powered on, is further configured to control the connection between the first end and the second end of the activation circuit to remain continuously conductive.

3. The first electronic device according to claim 1 or 2, wherein the activation circuit comprises a first MOS transistor and a first sensor, wherein a drain of the first MOS transistor is the first end, a source of the first MOS transistor is the second end, one end of the first sensor is connected to a gate of the first MOS transistor, and the other end of the first sensor is grounded;
the first sensor is configured to detect whether the user wears the first electronic device or whether the user prepares to wear the first electronic device;
the first sensor switches from being cut off to being conducted when detecting that the user wears the first electronic device or the user prepares to wear the first electronic device; and
when the first sensor is cut off, the first MOS transistor is cut off, and when the first sensor is conducted, the first MOS transistor is conducted.

4. The first electronic device according to claim 3, wherein the activation circuit further comprises a first resistor, wherein one end of the first resistor is connected to the source of the first MOS transistor, and the other end of the first resistor is connected to the gate of the first MOS transistor.

5. The first electronic device according to claim 3 or 4, wherein the activation circuit, the power management module, the battery, and the data processing module are all located in a first component of the first electronic device, the first component is located within a second component, and the first component is detached from the second component when the user wears the first electronic device;
the first sensor is an optical sensor, the first sensor is disposed on a side facing the second component when the first component is located within the second component, and the first sensor is configured to sense changes in illumination; and
the first sensor switches from being cut off to being conducted when sensing that light intensity is greater than a first threshold.

6. The first electronic device according to claim 3 or 4, wherein the activation circuit, the power management module, the battery, and the data processing module are all located in a first component of the first electronic device, the first component is covered by a third component, the third component is opaque, and the user removes the third component when preparing to wear the first electronic device;
the first sensor is an optical sensor, the first sensor is disposed on a side facing the third component, and the first sensor is configured to sense changes in illumination; and
the first sensor switches from being cut off to being conducted when sensing that light intensity is greater than a first threshold.

7. The first electronic device according to claim 3 or 4, wherein the activation circuit, the power management module, the battery, and the data processing module are all located in a first component of the first electronic device, the first component is located within a second component, and the first component is detached from the second component when the user wears the first electronic device;
the first sensor is a magnetic sensor, the second component comprises a magnet, and the first sensor is configured to sense changes in a magnetic field; and
the first sensor switches from being cut off to being conducted when sensing that magnetic field strength of the magnet is less than a second threshold.

8. The first electronic device according to claim 3 or 4, wherein the activation circuit, the power management module, the battery, and the data processing module are all located in a first component of the first electronic device, the first component is covered by a third component, and the user removes the third component when preparing to wear the first electronic device;
the first sensor is a magnetic sensor, the third component comprises a magnet, and the first sensor is configured to sense changes in a magnetic field; and
the first sensor switches from being cut off to being conducted when sensing that magnetic field strength of the magnet is less than a second threshold.

9. The first electronic device according to any one of claims 3 to 8, wherein the activation circuit further comprises a second MOS transistor, a gate of the second MOS transistor is connected to the data processing module, a source of the second MOS transistor is grounded, and a drain of the second MOS transistor is connected to the gate of the first MOS transistor;
the data processing module, when powered on, outputs a first voltage to the gate of the second MOS transistor; and
when the gate of the second MOS transistor receives the first voltage, the second MOS transistor switches from being cut off to being conducted, so that the first MOS transistor is continuously conducted.

10. The first electronic device according to any one of claims 1 to 9, wherein the activation circuit is configured to detect whether the user wears the first electronic device, and the first electronic device further comprises a timing module and a communication module, wherein
the timing module is configured to start timing after the data processing module is powered on, wherein duration of timing of the first electronic device is a length of time elapsed since the user started wearing the first electronic device; and the communication module is configured to: send the data of the body indicator to a second electronic device after establishing a communication connection to the second electronic device, and send, to the second electronic device, first duration obtained by the first electronic device through timing, wherein the first duration is related to a first time, and the first time is a time at which the second electronic device starts to display the data of the body indicator.

11. The first electronic device according to any one of claims 1 to 9, wherein the activation circuit is configured to detect whether the user prepares to wear the first electronic device, and the first electronic device further comprises a timing module, a communication module, and a second sensor;
the second sensor is configured to collect the data used to reflect the body indicator after the data processing module is powered on; and
the timing module is configured to start timing when the data collected by the second sensor meets a first preset condition, wherein duration of timing of the first electronic device is a length of time elapsed since the user started wearing the first electronic device; and the communication module is configured to: send the data of the body indicator to a second electronic device after establishing a communication connection to the second electronic device, and send, to the second electronic device, first duration obtained by the first electronic device through timing, wherein the first duration is related to a first time, and the first time is a time at which the second electronic device starts to display the data of the body indicator.

12. The first electronic device according to any one of claims 1 to 11, wherein the activation circuit is configured to detect whether the user prepares to wear the first electronic device, and the first electronic device further comprises a second sensor and an output module;
the second sensor is configured to collect the data used to reflect the body indicator after the data processing module is powered on;
the output module is configured to output first prompt information when the data collected by the second sensor meets a second preset condition, wherein the first prompt information prompts the user to wear the first electronic device in a timely manner; and
the output module is further configured to output second prompt information when the data collected by the second sensor meets a third preset condition, wherein the second prompt information prompts a wear failure of the first electronic device to the user.

13. The electronic device according to any one of claims 1 to 12, wherein
the first electronic device is a continuous glucose monitoring CGM device, and the body indicator is blood glucose; or
the first electronic device is a continuous ketone monitoring CKM device, and the body indicator is blood ketone; or
the first electronic device is a continuous lactate monitoring CLM device, and the body indicator is lactate; or
the first electronic device is an electrocardiogram patch, and the body indicator is an electrocardiogram signal.

14. A device activation method, wherein the method is applied to a first electronic device, the first electronic device is configured to measure a body indicator of a user, the first electronic device comprises an activation circuit, a power management module, a battery, and a data processing module, the activation circuit comprises a first end and a second end, the first end is connected to the power management module, the second end is connected to the battery, and the power management module is connected to the data processing module, and the method comprises:
determining, by the first electronic device by using the activation circuit, whether the user wears the first electronic device or whether the user prepares to wear the first electronic device;
switching, by the first electronic device by using the activation circuit when determining that the user wears the first electronic device or the user prepares to wear the first electronic device, a connection between the first end and the second end from being cut off to being conducted;
enabling, by the first electronic device after the connection between the first end and the second end is conducted, the battery to supply power to the data processing module by using the power management module; and
determining, by the first electronic device by using the data processing module, the body indicator of the user based on data obtained by the first electronic device.

15. The method according to claim 14, wherein the method further comprises:
controlling, by the first electronic device, the connection between the first end and the second end of the activation circuit to be continuously conducted in a power-on condition of the data processing module.

16. The method according to 14 or 15, wherein the activation circuit comprises a first MOS transistor and a first sensor, wherein a drain of the first MOS transistor is the first end, a source of the first MOS transistor is the second end, one end of the first sensor is connected to a gate of the first MOS transistor, and the other end of the first sensor is grounded;
detecting, by the first electronic device by using the activation circuit, whether the user wears the first electronic device or whether the user prepares to wear the first electronic device specifically comprises:
detecting, by the first electronic device by using the first sensor, whether the user wears the first electronic device or whether the user prepares to wear the first electronic device;
switching, by the first sensor, from being cut off to being conducted when detecting that the user wears the first electronic device or the user prepares to wear the first electronic device; and
when the first sensor is cut off, the first MOS transistor being cut off, and when the first sensor is conducted, the first MOS transistor being conducted.

17. The method according to claim 16, wherein the activation circuit, the power management module, the battery, and the data processing module are all located in a first component of the first electronic device, the first component is located within a second component, and the first component is detached from the second component when the user wears the first electronic device;
the first sensor is an optical sensor, and the first sensor is disposed on a side facing the second component when the first component is located within the second component; and
detecting, by the first electronic device by using the first sensor, whether the user wears the first electronic device specifically comprises:
detecting, by the first electronic device based on an illumination change sensed by the first sensor, whether the user wears the first electronic device; and
switching, by the first sensor, from being cut off to being conducted when sensing that light intensity is greater than a first threshold.

18. The method according to claim 16, wherein the activation circuit, the power management module, the battery, and the data processing module are all located in a first component of the first electronic device, the first component is covered by a third component, the third component is opaque, and the user removes the third component when preparing to wear the first electronic device;
the first sensor is an optical sensor, and the first sensor is disposed on a side facing the third component; and
detecting, by the first electronic device by using the first sensor, whether the user prepares to wear the first electronic device specifically comprises:
detecting, by the first electronic device based on an illumination change sensed by the first sensor, whether the user prepares to wear the first electronic device; and
switching, by the first sensor, from being cut off to being conducted when sensing that light intensity is greater than a first threshold.

19. The method according to claim 16, wherein the activation circuit, the power management module, the battery, and the data processing module are all located in a first component of the first electronic device, the first component is located within a second component, and the first component is detached from the second component when the user wears the first electronic device;
the first sensor is a magnetic sensor, and the second component comprises a magnet; and
detecting, by the first electronic device by using the first sensor, whether the user wears the first electronic device specifically comprises:
detecting, by the first electronic device based on changes in a magnetic field sensed by the first sensor, whether the user wears the first electronic device; and
switching, by the first sensor, from being cut off to being conducted when sensing that magnetic field strength of the magnet is less than a second threshold.

20. The method according to claim 16, wherein the activation circuit, the power management module, the battery, and the data processing module are all located in a first component of the first electronic device, the first component is covered by a third component, and the user removes the third component when preparing to wear the first electronic device;
the first sensor is a magnetic sensor, and the third component comprises a magnet; and
detecting, by the first electronic device by using the first sensor, whether the user prepares to wear the first electronic device specifically comprises:
detecting, by the first electronic device based on changes in a magnetic field sensed by the first sensor, whether the user prepares to wear the first electronic device; and
switching, by the first sensor, from being cut off to being conducted when sensing that magnetic field strength of the magnet is less than a second threshold.

21. The method according to any one of claims 16 to 20, wherein the activation circuit further comprises a second MOS transistor, a gate of the second MOS transistor is connected to the data processing module, a source of the second MOS transistor is grounded, and a drain of the second MOS transistor is connected to the gate of the first MOS transistor, and after enabling, by the first electronic device, the battery to supply the power to the data processing module by using the power management module, the method further comprises:
when the data processing module powered on, outputting, by the first electronic device, a high voltage to the gate of the second MOS transistor using the data processing module; and
when the gate of the second MOS transistor receives the high voltage, enabling, by the first electronic device by using the second MOS transistor, the first MOS transistor to be continuously conducted, so that the second MOS transistor switches from being cut off to being conducted when the gate of the second MOS transistor receives the high voltage.

22. The method according to any one of claims 14 to 21, wherein the first electronic device detects, by using the activation circuit, whether the user wears the first electronic device, and after enabling, by the first electronic device, the battery to supply the power to the data processing module by using the power management module, the method further comprises:
starting, by the first electronic device, timing, wherein duration of timing of the first electronic device is a length of time elapsed since the user started wearing the first electronic device; and
sending, by the first electronic device, the data of the body indicator to a second electronic device after establishing a communication connection to the second electronic device, and sending, to the second electronic device, first duration obtained by the first electronic device through timing, wherein the first duration is related to a first time, and the first time is a time at which the second electronic device starts to display the data of the body indicator.

23. The method according to any one of claims 14 to 22, wherein the first electronic device further comprises a second sensor, the first electronic device detects, by using the activation circuit, whether the user prepares to wear the first electronic device, and after enabling, by the first electronic device, the battery to supply the power to the data processing module by using the power management module, the method further comprises:
collecting, by the first electronic device by using the second sensor, the data used to reflect the body indicator;
starting, by the first electronic device, timing when the data collected by the second sensor meets a first preset condition, wherein duration of timing of the first electronic device is a length of time elapsed since the user started wearing the first electronic device; and
sending, by the first electronic device, the data of the body indicator to a second electronic device after establishing a communication connection to the second electronic device, and sending, to the second electronic device, first duration obtained by the first electronic device through timing, wherein the first duration is related to a first time, and the first time is a time at which the second electronic device starts to display the body indicator.

24. The method according to any one of claims 14 to 23, wherein the first electronic device detects, by using the activation circuit, whether the user prepares to wear the first electronic device, the first electronic device further comprises a second sensor, and the method further comprises:
collecting, by the first electronic device by using the second sensor, the data used to reflect the body indicator after the data processing module is powered on;
outputting, by the first electronic device, first prompt information when the data collected by the second sensor meets a second preset condition, wherein the first prompt information prompts the user to wear the first electronic device in a timely manner; and
outputting, by the first electronic device, second prompt information when the data collected by the second sensor meets a third preset condition, wherein the second prompt information prompts a wear failure of the first electronic device to the user.

25. The method according to any one of claims 14 to 24, wherein
the first electronic device is a continuous glucose monitoring CGM device, and the body indicator is blood glucose; or
the first electronic device is a continuous ketone monitoring CKM device, and the body indicator is blood ketone; or
the first electronic device is a continuous lactate monitoring CLM device, and the body indicator is lactate; or
the first electronic device is an electrocardiogram patch, and the body indicator is an electrocardiogram signal.

26. An electronic device, comprising a first electronic device, a second component, and/or a third component, wherein
if the electronic device comprises the first electronic device and the second component, a first component of the first electronic device is located in the second component;
if the electronic device comprises the first electronic device and the third component, the third component covers the first component in the first electronic device; or
if the electronic device comprises the first electronic device, the second component, and the third component, the first component of the first electronic device is located in the second component, and the third component covers a transmission outlet of the second component and is configured to cover the first component, wherein
the first electronic device is the first electronic device according to any one of claims 1 to 13.

27. An electronic device, comprising a memory, one or more processors, and one or more programs, wherein when the one or more processors execute the one or more programs, the electronic device is enabled to implement the method according to any one of claims 14 to 25.

28. A computer-readable storage medium, comprising instructions, wherein when the instructions are run on an electronic device, the electronic device is enabled to perform the method according to any one of claims 14 to 25.
